# EUROPEAN PATENT APPLICATION

(11) **EP 3 868 475 A1**
(43) Date of publication of application: **25.08.2021**
(21) Application number: 21169297.5
(22) Date of filing: 05.10.2017
(51) Int. Cl.: B01L 3/00, B01F 13/02

(54) **METHOD FOR TESTING A SAMPLE**

(30) Priority: 07.10.2016 EP 16020372
(62) Divisional of application: 17784847.0
(71) Applicant: Boehringer Ingelheim Vetmedica GmbH, 55216 Ingelheim am Rhein (DE)
(72) Inventor: SCHMOLKE, Hannah, 55216 Ingelheim am Rhein (DE); KRONSBEIN, Matthias, 66482 Zweibruecken (DE); SCHOEDER, Heinz, 55216 Ingelheim am Rhein (DE); WEBER, Lutz, 66482 Zweibruecken (DE)
(74) Representative: Von Rohr Patentanwälte Partnerschaft mbB

(57) **Abstract**

A method for testing a biological sample is proposed, wherein the sample is divided into a plurality of sample portions, the sample portions being fed sequentially to a sensor arrangement in order to bond the analytes of the sample portions to corresponding capture molecules.

## Description

The present invention relates to a method according to the preamble of claim 1.

Preferably, the present invention deals with analysing and testing a sample, in particular from a human or animal, particularly preferably for analytics and diagnostics, for example with regard to the presence of diseases and/or pathogens and/or for determining blood counts, antibodies, hormones, steroids or the like. Therefore, the present invention is in particular within the field of bioanalytics. A food sample, environmental sample or another sample may optionally also be tested, in particular for environmental analytics or food safety and/or for detecting other substances.

Preferably, by means of the present invention, at least one analyte (target analyte) of a sample can be determined, detected or identified. In particular, the sample can be tested for qualitatively or quantitatively determining at least one analyte, for example in order for it to be possible to detect or identify a disease and/or pathogen.

Preferably, by means of the present invention, nucleic-acid sequences, in particular DNA sequences and/or RNA sequences, can be determined, detected or identified as analytes of a sample, or proteins, in particular antigens and/or antibodies, can be determined, detected or identified as analytes of the sample. More particularly preferably, the present invention deals with systems, devices and other apparatuses for carrying out a nucleic-acid assay for detecting or identifying a nucleic-acid sequence or a protein assay for detecting or identifying a protein.

The present invention deals in particular with what are known as point-of-care systems, i.e. in particular with mobile systems, devices and other apparatuses, and deals with methods for carrying out tests on a sample at the sampling site and/or separately or away from a central laboratory or the like. Preferably, point-of-care systems can be operated autonomously of or independently from a mains network for supplying electrical power.

US 5,096,669 discloses a point-of-care system for testing a biological sample, in particular a blood sample. The system comprises a single-use cartridge and an analysis device. Once the sample has been received, the cartridge is inserted into the analysis device in order to carry out the test. The cartridge comprises a microfluidic system and a sensor apparatus comprising electrodes, which apparatus is calibrated by means of a calibration liquid and is then used to test the sample.

Furthermore, WO 2006/125767 A1 discloses a point-of-care system for integrated and automated DNA or protein analysis, comprising a single-use cartridge and an analysis device for fully automatically processing and evaluating molecular-diagnostic analyses using the single-use cartridge. The cartridge is designed to receive a sample, in particular blood, and in particular allows cell disruption, PCR and detection of PCR amplification products, which are bonded to capture molecules and provided with a label enzyme, in order for it to be possible to detect bonded PCR amplification products or nucleic-acid sequences as target analytes in what is known as a redox cycling process.

DE 10 2014 200 483 A1 discloses a microfluidic chip for PCR and analyzing a biological sample,. An array chamber for analyzing can be flushed. A division of the sample into a plurality of sample portions is not disclosed in the sample.

US 2013/0280698 A1 discloses a device for simultaneously conducting multiple assays on a liquid sample. The sample is divided into several portions which are then transferred to separate assay chambers for simultaneously conducting separate assays on the sample portions.

WO 2007/089587 A2 discloses a microfluidic device for analysis of interactions between molecules. The device comprises a plurality of unit cells, each unit cell comprising a reaction chamber with a reagent. The unit cells can each contain different reagents. A parallel detection of molecule interactions occurring in the different unit cells is possible.

EP 2 143 491 A1 discloses a device for analyzing a chemical or biological sample. The device has a plurality of discs which can be rotated relative to one another. By rotating, different chambers and channels of the device can be fluidically connected to form different loops. The device comprises ten separate PCR chambers. Thus, ten independent reactions can be run simultaneously.

WO 2013/086505 A1 relates to an integratesd organ-on-chip system with a plurality of cartridges, wherein each cartridge simulates an individual organ. Valves are provided on the cartridges so that different fluidic connections, for example inlets and outlets, can be fluidically connected. The cartridges can be arranged in an array for analysing a plurality of samples individually or simultaneously. It is not disclosed to divide a sample into different portions.

The problem addressed by the present invention is to provide an improved method and an improved cartridge for testing a sample, which preferably allow or facilitate comprehensive, efficient, rapid, reliable, hygienic, robust and/or precise testing of the sample and/or a cost-effective and/or compact design of the cartridge.

The above problem is solved by a method according to claim 1. Advantageous developments are the subject of the dependent claims.

In the proposed method for testing an in particular biological sample, a sample is conveyed or pumped through a fluid system having a plurality of channels and cavities in a cartridge, in particular by means of a pump apparatus of the cartridge, the sample preferably being pretreated in the cartridge and analytes of the sample being identified or detected by means of a sensor arrangement and/or sensor apparatus, in particular electrochemically and/or by redox cycling.

One aspect of the present invention involves feeding the sample to the sensor arrangement or sensor apparatus for detecting analytes of the sample in a first conveying direction, in particular in order to bond the analytes to corresponding capture molecules, and, in particular after the analytes have bonded to the corresponding capture molecules, carrying the sample or sample residue away from the sensor arrangement or sensor apparatus in a second conveying direction which is opposite to the first conveying direction.

Preferably, the sample is fed to the sensor arrangement or sensor apparatus and carried away from the sensor arrangement or sensor apparatus via the same opening and/or at least in portions via the same channel. Advantageously, a simple construction of the fluid system is thus made possible, contamination of other and/or several channels and/or channel portions by the sample is prevented, and/or it is possible to immediately flush and/or empty the channels and/or channel portions used.

According to another aspect of the present invention, which can also be implemented independently, the sample, in particular in the cartridge and/or after the sample is placed into the cartridge, is divided into a plurality of sample portions, preferably at least two or three portions, preferably the sample portions each being conveyed in the fluid system individually and/or independently from one another and/or sequentially, in particular being pretreated or prepared and/or fed to the (common) sensor arrangement or sensor apparatus, or to a common sensor compartment of the sensor arrangement. This makes it possible to carry out different tests and/or to prepare or pretreat the sample portions for the tests, which are in particular different, in a targeted and/or different manner.

Preferably, the sample is divided into sample portions that are at least substantially the same size and/or sample portions that have at least substantially the same volume. However, variants of the method are also possible in which the sample is divided into sample portions of different sizes.

The sample is preferably divided into sample portions by accordingly activating valves and/or a pump apparatus of the cartridge. In particular, the sample is divided into a plurality of sample portions by removing the sample from a cavity in a selective and/or metered manner.

Particularly preferably, the sample portions are each handled individually and/or conveyed individually in the fluid system. In particular, the sample portions are each conveyed to the sensor apparatus individually and are each conveyed away from the sensor arrangement or sensor apparatus individually.

A particularly preferred aspect of the present invention involves the sample portions being fed to the sensor arrangement or sensor apparatus sequentially and/or in succession and/or in the first conveying direction, in particular in order to sequentially bond the analytes of the sample portions to the corresponding capture molecules of the sensor arrangement or sensor apparatus, and, subsequently and/or after the analytes have bonded to the corresponding capture molecules, to remove or carry away said analytes from the sensor arrangement or sensor apparatus sequentially and/or in the second conveying direction, which is opposite to the first conveying direction, in particular in order to collect the sample portions in a (common) collection cavity. This results in corresponding advantages.

The term "conveying direction" is preferably understood to mean the direction in which the fluid is conveyed in the cartridge. Particularly preferably, the conveying direction is the direction in which the fluid is conveyed in the pump apparatus and/or directly upstream of and/or at the inlet of, or downstream of and/or at the outlet of the sensor arrangement or sensor apparatus. In particular, within the meaning of the present invention, the conveying direction is determined by the operation or actuation of the pump apparatus and/or is changed or reversed by accordingly activating the pump apparatus, in particular by changing the rotational direction of a pump drive. The conveying direction may, however, also be determined or changed by accordingly activating or actuating the valves, in particular without changing the operation of the pump apparatus, in particular the rotational direction of the pump drive.

In the proposed method, a sensor cover that is in particular flexible or movable at least in part is preferably moved relative to the sensor apparatus and/or lowered onto the sensor apparatus for improved detection.

By actuating or lowering the sensor cover when detecting or in order to detect the (bonded) analytes, the sensor fields of the sensor apparatus and/or sensor array are sealed and/or fluidically separated, in particular such that an exchange of substances and/or chemical crosstalk between the sensor fields is minimised or prevented. In this way, misallocations of measurements to the wrong sensor fields and/or measurement errors resulting from misallocations or from chemical crosstalk between adjacent sensor fields are prevented or at least minimised.

According to another aspect of the present invention, which can also be implemented independently, the sensor arrangement or sensor apparatus is pretreated and/or flushed, preferably with a fluid, in particular a wash buffer and/or a reagent, for the detection of the (bonded) analytes and/or immediately before detection of the (bonded) analytes, the sensor cover preferably being actuated and/or lowered onto the sensor apparatus for and/or during the pretreatment, in particular multiple times and/or both for pretreatment and for detection.

By actuating and/or lowering the sensor cover during the pretreatment of the sensor arrangement or sensor apparatus, in particular when flushing the sensor arrangement or sensor apparatus with a wash buffer, individual sensor fields and/or sensor cavities of the sensor apparatus are flushed particularly effectively and any bubbles, remnants or the like are removed. In particular, by lowering the sensor cover, the pressure in a sensor compartment and/or in the sensor fields and/or the turbulence of the flow in a sensor compartment and/or in the sensor fields is increased at least temporarily. In this way, the pretreatment of the sensor apparatus is optimised and/or any measurement inaccuracies and/or the risk of measurement errors caused by bubbles, remnants or the like are reduced. Preferably, the sensor cover is actuated multiple times and/or is lowered onto the sensor apparatus multiple times, and is also raised again at least once. In particular, the sensor cover is used or actuated multiple times during the pretreatment of the analytes or sensor arrangement.

Particularly preferably, the sensor arrangement or sensor apparatus, or the bonded analytes, is/are prepared or pretreated in a plurality of method steps, in particular for the (subsequent) detection of the bonded analytes, the sensor cover preferably being actuated and/or lowered multiple times, in particular in some or all of the method steps for pretreatment.

Within the meaning of the present invention, the term "pretreatment" is understood to mean one or more method steps which are required for identifying or detecting the (bonded) analytes and/or which are carried out (immediately) before the (bonded) analytes are (actually) detected. The pretreatment of the sensor arrangement or sensor apparatus preferably includes flushing the sensor arrangement, in particular the sensor compartment, particularly preferably by means of a wash buffer, and/or flushing or loading the sensor arrangement or sensor compartment with one or more reagents, in particular with detector molecules and/or a substrate, particularly preferably for carrying out the reactions necessary for the detection.

In the proposed method, the sample is preferably placed into a cartridge, for example by means of a pipette, and the cartridge containing the sample is received by and/or inserted into an analysis device for testing the sample.

According to another aspect of the present invention, which can also be implemented independently, different fluidic circuits are formed or activated in the cartridge - in particular by selectively actuating or activating valves in the cartridge -, preferably in order to carry out the proposed method and/or with the sample or sample portions and/or a fluid being conveyed in all or each of the fluidic circuits by means of a (common) pump apparatus of the cartridge. In particular, a (common) pump apparatus of the cartridge is used to convey the sample or sample portions and/or a fluid for the individual method steps of the proposed method and/or in different fluidic circuits. This allows or facilitates a particularly compact design of the cartridge.

The proposed analysis system for testing an in particular biological sample preferably comprises a proposed analysis device and a proposed cartridge for testing the sample, the cartridge preferably being designed for receiving the sample and the analysis device preferably being designed for receiving the cartridge. The proposed analysis system and/or the proposed cartridge are designed in particular for carrying out the proposed method.

The analysis system is preferably portable, mobile and/or is a point-of-care system and/or can be used in particular at the sampling site and/or away from a central laboratory and/or can be operated autonomously and/or independently of the mains, in particular independently of a mains power supply, for example by accumulators, batteries and/or other power storage means.

The term "analysis device" is preferably understood to mean an instrument which is in particular mobile and/or can be used on site, and/or which is designed to chemically, biologically and/or physically test and/or analyse a sample or a component thereof, preferably in and/or by means of a cartridge. In particular, the analysis device controls the pretreatment and/or testing of the sample in the cartridge.

Particularly preferably, the analysis device is designed to receive the cartridge or to connect said cartridge electrically, thermally and/or pneumatically.

The term "cartridge" is preferably understood to mean a structural apparatus or unit designed to receive, to store, to physically, chemically and/or biologically treat and/or prepare and/or to measure a sample, preferably in order to make it possible to detect, identify or determine at least one analyte, in particular a protein and/or a nucleic-acid sequence, of the sample.

In particular, within the meaning of the present invention, a cartridge is designed to be at least substantially planar and/or card-like, in particular is designed as a (micro)fluidic card and/or is designed as a main body or container that can preferably be closed and/or said cartridge can be inserted and/or plugged into a proposed analysis device when it contains the sample.

A cartridge within the meaning of the present invention preferably comprises a fluid system having a plurality of channels, cavities and/or valves for controlling the flow through the channels and/or cavities.

Preferably, the analysis system, in particular the cartridge, comprises a pump apparatus for conveying the sample and/or a fluid, in particular a reagent and/or wash buffer, in the fluid system.

According to another aspect of the present invention, which can also be implemented independently, one of the cavities is designed as a collection cavity, both the collection cavity and pump apparatus and at least one other of the cavities, in particular a storage cavity containing a fluid, such as a reagent and/or a wash buffer, being interconnected or interconnectable in a fluidic circuit in order to convey a fluid out of the other cavity and/or to displace said fluid out by means of another fluid taken from the collection cavity, in particular a gas, and to feed said fluid to a sensor arrangement. In this way, it is possible to prevent vacuums in the fluid system.

According to another aspect of the present invention, which can also be implemented independently, the cartridge comprises a receiving cavity for receiving the sample and a mixing cavity for mixing the sample with a reagent, the receiving cavity, the mixing cavity and the pump apparatus being interconnected or interconnectable in a first fluidic circuit, such that the sample can be conveyed from the receiving cavity into the mixing cavity by means of the pump apparatus, and the mixing cavity and the pump apparatus, in particular without the receiving cavity, being interconnected or interconnectable in a second fluidic circuit that is different from the first fluidic circuit, such that a gas can be drawn out of the mixing cavity at the top by means of the pump apparatus in the normal operating position of the cartridge and can be conveyed or blown into the mixing cavity at the bottom in order to mix the sample with a reagent, in particular by turbulence and/or by means of the rising gas. Advantageously, the pump apparatus can be used both for conveying and for assisting in the pretreatment of the sample.

The analysis system, in particular the cartridge, preferably comprises a sensor arrangement or sensor apparatus for identifying or detecting analytes of the sample, the sensor arrangement or sensor apparatus preferably being provided with capture molecules for capturing and/or bonding the analytes.

The sensor apparatus is preferably designed to carry out a protein assay and/or a nucleic-acid assay. In particular, the sensor apparatus comprises capture proteins as capture molecules for detecting or identifying a target protein and/or comprises capture nucleic-acid sequences as capture molecules for detecting or identifying a target nucleic-acid sequence, in particular in order to bond corresponding target proteins to the capture proteins and to bond corresponding target nucleic-acid sequences to the capture nucleic-acid sequences.

The above-mentioned aspects and features of the present invention and the aspects and features of the present invention that will become apparent from the claims and the following description can in principle be implemented independently from one another, but also in any combination or order.

Other aspects, advantages, features and properties of the present invention will become apparent from the claims and the following description of a preferred embodiment with reference to the drawings, in which:
Fig. 1 is a schematic view of a proposed analysis system comprising a proposed analysis device and a proposed cartridge received in the analysis device;
Fig. 2 is a schematic view of the proposed cartridge;
Fig. 3 is a schematic sectional view of a sensor arrangement of the analysis system and/or of the cartridge with the sensor cover moved away and during pretreatment;
Fig. 4 is a schematic sectional view of the sensor arrangement according to Fig. 3 with the sensor cover lowered and during detection;
Fig. 5 is a schematic view of the cartridge when the sample is being divided into sample portions;
Fig. 6 is a schematic view of the cartridge when one of the sample portions is being fed to the sensor arrangement;
Fig. 7 is a schematic view of the cartridge when one of the sample portions is being conveyed away from the sensor arrangement; and
Fig. 8 is a schematic view of the cartridge when the sensor arrangement is being flushed.

In the Figures, which are only schematic and sometimes not to scale, the same reference signs are used for the same or similar parts and components, corresponding or comparable properties and advantages being achieved even if these are not repeatedly described.

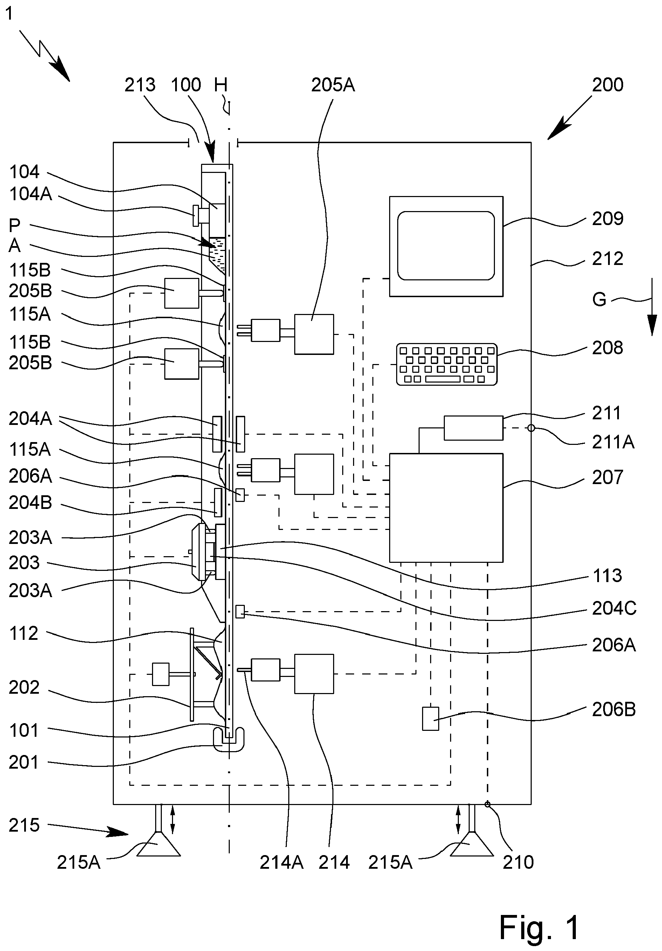

Fig. 1 is a highly schematic view of a proposed analysis system 1 and analysis device 200 for testing an in particular biological sample P, preferably by means of or in an apparatus or cartridge 100.

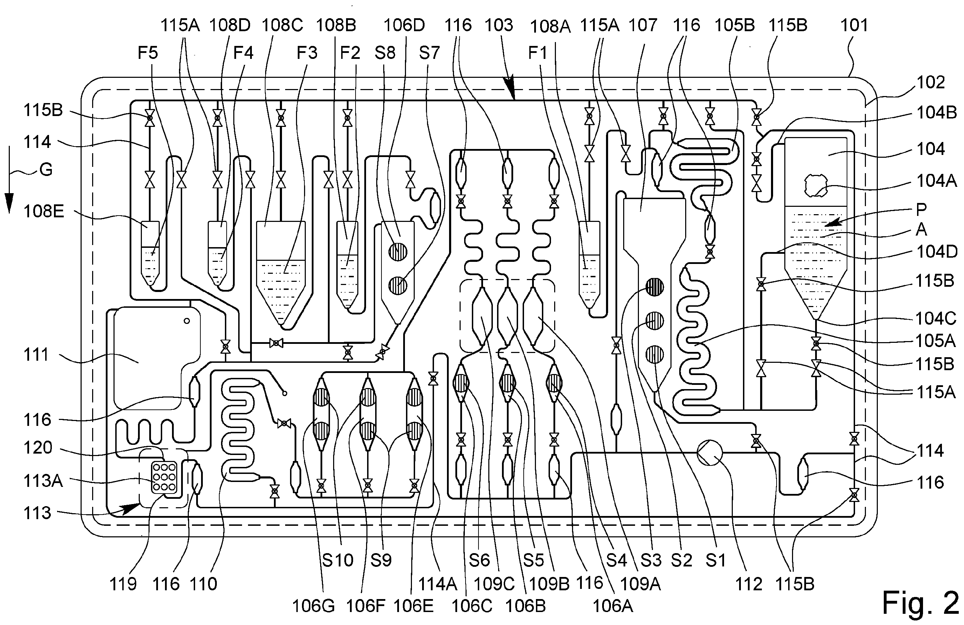

Fig. 2 is a schematic view of a preferred embodiment of the proposed apparatus or cartridge 100 for testing the sample P. The apparatus or cartridge 100 in particular forms a handheld unit, and in the following is merely referred to as a cartridge 100.

The term "sample" is preferably understood to mean the sample material to be tested, which is in particular taken from a human or animal. In particular, within the meaning of the present invention, a sample P is a fluid, such as saliva, blood, urine or another liquid, preferably from a human or animal, or a component thereof. Within the meaning of the present invention, a sample P may be pretreated or prepared if necessary, or may come directly from a human or animal or the like, for example. A food sample, environmental sample or another sample may optionally also be tested, in particular for environmental analytics, food safety and/or for detecting other substances, preferably natural substances, but also biological or chemical warfare agents, poisons or the like.

A sample P within the meaning of the present invention preferably contains one or more analytes A, it preferably being possible for the analytes A to be identified or detected, in particular qualitatively and/or quantitatively determined. Particularly preferably, within the meaning of the present invention, a sample P has target nucleic-acid sequences as the analytes A, in particular target DNA sequences and/or target RNA sequences, and/or target proteins as the analytes A, in particular target antigens and/or target antibodies. Particularly preferably, at least one disease and/or pathogen can be identified or detected in the sample P by qualitatively and/or quantitatively determining the analytes A.

Preferably, the analysis system 1 or analysis device 200 controls the testing of the sample P in particular in or on the cartridge 100 and/or is used to evaluate the testing or the collection, processing and/or storage of measured values from the test.

By means of the proposed analysis system 1 or analysis device 200 or by means of the cartridge 100 and/or using the proposed method for testing the sample P, preferably an analyte A of the sample P, in particular a (certain) nucleic-acid sequence or target nucleic-acid sequence and/or a (certain) protein or target protein, or particularly preferably a plurality of analytes A of the sample P, can be determined, detected or identified. Said analytes are in particular detected or identified and/or measured not only qualitatively, but particularly preferably also quantitatively.

Therefore, the sample P can in particular be tested for qualitatively or quantitatively determining at least one analyte A, for example in order for it to be possible to detect or identify a disease and/or pathogen or to determine other values, which are important for diagnostics, for example.

Particularly preferably, a molecular-biological test is made possible by means of the analysis system 1 and/or analysis device 200 and/or by means of the cartridge 100.

Particularly preferably, a nucleic-acid assay for detecting or identifying a target nucleic-acid sequence, in particular a target DNA sequence and/or a target RNA sequence, and/or a protein assay for detecting or identifying a target protein, in particular a target antigen and/or target antibody, are made possible or are carried out.

The term "assay" is preferably understood to mean an in particular molecular-biological test for detecting or identifying at least one analyte A in a sample P. In particular, at least one analyte A in a sample P can be qualitatively or quantitatively detected or identified by means of an assay or by carrying out an assay. A plurality of method steps are preferably required to (fully) carry out an assay. Preferably, within the meaning of the present invention, when carrying out an assay, a sample P is pretreated with one or more reagents and the pretreated sample P is tested, in particular at least one analyte A in the sample P being detected or identified. Within the meaning of the present invention, an assay is in particular an immunoassay or protein assay for detecting or identifying a target protein, in particular a target antigen and/or target antibody, and/or a nucleic-acid assay for detecting or identifying a target nucleic-acid sequence, in particular a target DNA sequence and/or target RNA sequence.

Preferably, the sample P or individual components of the sample P or analyte A can be amplified if necessary, in particular by means of PCR, and tested, detected or identified in the analysis system 1 or analysis device 200 or in the cartridge 100, and/or for the purpose of carrying out the nucleic-acid assay. Preferably, amplification products of the analyte A or analytes A are thus produced.

In the following, further details are first given on a preferred construction of the cartridge 100, with features of the cartridge 100 preferably also directly representing features of the analysis system 1, in particular even without any further explicit explanation.

The cartridge 100 is preferably at least substantially planar, plate-shaped, flat and/or card-like.

The cartridge 100 preferably comprises an in particular at least substantially planar, flat, plate-shaped and/or card-like main body or support 101, the main body or support 101 in particular being made of and/or injection-moulded from plastics material, particularly preferably polypropylene.

The cartridge 100 preferably comprises at least one film or cover 102 for covering the main body 101 and/or cavities and/or channels formed therein at least in part, in particular on the front side, and/or for forming valves or the like, as shown by dashed lines in Fig. 2.

The analysis system 1 or cartridge 100 or the main body 101 thereof, in particular together with the cover 102, preferably forms and/or comprises a fluidic system 103, referred to in the following as the fluid system 103.

The cartridge 100, the main body 101 and/or the fluid system 103 or its main plane are/is preferably at least substantially vertically oriented in the operating position and/or during the test, in particular in the analysis device 200, as shown schematically in Fig. 1.

Preferably, the cartridge 100, in particular the main body 101, has a main plane of extension H, the main plane of extension H preferably extending at least substantially vertically and/or in parallel with gravity G in the normal operating position and/or when the cartridge 100 is received.

The cartridge 100 and/or the fluid system 103 preferably comprises a plurality of cavities, in particular at least one receiving cavity 104, at least one metering cavity 105, at least one intermediate cavity 106, at least one mixing cavity 107, at least one storage cavity 108, at least one reaction cavity 109, at least one intermediate temperature-control cavity 110 and/or at least one collection cavity 111, the cavities preferably being fluidically interconnected by a plurality of channels.

Within the meaning of the present invention, channels are preferably elongate forms for conducting a fluid in a main flow direction or conveying direction, the forms preferably being closed transversely, in particular perpendicularly, to the main flow direction and/or longitudinal extension, preferably on all sides.

In particular, the main body 101 comprises elongate notches, recesses, depressions or the like, which are closed at the sides by the cover 102 and form channels within the meaning of the present invention.

Within the meaning of the present invention, cavities or chambers are preferably formed by recesses, depressions or the like in the cartridge 100 or support 101, which are closed or covered by the cover 102, in particular at the sides. The space enclosed by each cavity is preferably fluidically linked by means of the channels.

Within the meaning of the present invention, cavities preferably have a larger diameter and/or flow cross section and/or a larger volume than channels, preferably by at least a factor of 2, 3 or 4. In principle, however, cavities may in some cases also be elongate, in a similar manner to channels.

Preferably, within the meaning of the present invention, a cavity comprises at least two openings for the inflow and/or outflow of fluids and/or comprises an inlet and an outlet, in particular such that said fluid can flow through the cavities from the inlet to the outlet.

Preferably, several or all of the cavities are vertically oriented and/or are oriented such that fluid can flow through the cavities at least substantially vertically in the normal operating position of the cartridge 100.

Particularly preferably, several or all of the cavities, in particular the receiving cavity 104, the intermediate cavity/cavities 106, the mixing cavity 107, the storage cavity/cavities 108 and/or the reaction cavity/cavities 109, are elongate, the longitudinal extension of the cavities preferably extending at least substantially vertically, and/or in parallel with gravity G in the normal operating position of the cartridge 100.

Preferably, the inlet of several or all of the cavities is at the top in the normal operating position of the cartridge 100 and the outlet of several or all of the cavities is at the bottom in the normal operating position of the cartridge 100, in particular such that fluid can flow through or drain from some or all of the cavities, in particular the storage cavity/cavities 108, from the top to the bottom in the normal operating position and/or a fluid located in the cavities, in particular the storage cavity/cavities 108, can be removed and/or pumped out at the bottom. In this way, bubble formation and/or foaming of the fluids located in the cavities can be prevented. In particular, this prevents a gas, in particular air, from being conveyed out of the cavities.

The analysis system 1, in particular the cartridge 100 and/or the fluid system 103, also preferably comprises at least one pump apparatus 112 and/or at least one sensor arrangement or sensor apparatus 113.

In the example shown, the cartridge 100 or the fluid system 103 preferably comprises two metering cavities 105A and 105B, a plurality of intermediate cavities 106A to 106G, a plurality of storage cavities 108A to 108E and/or a plurality of reaction cavities 109, which can preferably be loaded separately from one another, in particular a first reaction cavity 109A, a second reaction cavity 109B and an optional third reaction cavity 109C, as can be seen in Fig. 2.

The metering cavities 105 are preferably designed to receive, to temporarily store and/or to meter the sample, and/or to pass on said sample in a metered manner. Particularly preferably, the metering cavities 105 have a diameter which is larger than that of the (adjacent) channels.

In the initial state of the cartridge 100 or when at the factory, the storage cavities 108 are preferably filled at least in part, in particular with a liquid such as a reagent, solvent or wash buffer.

The collection cavity 111 is preferably designed to receive larger quantities of fluids that are in particular used for the test, such as reagents, sample residues or the like. Preferably, in the initial state or when at the factory, the collection cavity 111 is empty or filled with gas, in particular air. The volume of the collection cavity 111 corresponds to or preferably exceeds the (cumulative) volume of the storage cavity/cavities 108 or the liquid content thereof and/or the volume of the receiving cavity 104 or the sample P received.

The reaction cavity/cavities 109 is/are preferably designed to allow a substance located in the reaction cavity 109 to react when an assay is being carried out, for example by being linked or coupled to apparatuses or modules of the analysis device 200.

The reaction cavity/cavities 109 is/are used in particular to carry out an amplification reaction, in particular PCR, or several, preferably different, amplification reactions, in particular PCRs. It is preferable to carry out several, preferably different, PCRs, i.e. PCRs having different primer combinations or primer pairs, in parallel and/or separately and/or in different reaction cavities 109.

To carry out the nucleic-acid assay, preferably target nucleic-acid sequences, as analytes A of the sample P, are amplified in the reaction cavity/cavities 109 by means of an amplification reaction, in particular in order to produce amplification products for the subsequent detection in the sensor arrangement or sensor apparatus 113.

Within the meaning of the present invention, amplification reactions are in particular molecular-biological reactions in which an analyte A, in particular a target nucleic-acid sequence, is amplified/copied and/or in which amplification products, in particular nucleic-acid products, of an analyte A are produced. Particularly preferably, PCRs are amplification reactions within the meaning of the present invention.

"PCR" stands for polymerase chain reaction and is a molecular-biological method by means of which certain analytes A, in particular portions of RNA or RNA sequences or DNA or DNA sequences, of a sample P are amplified, preferably in several cycles, using polymerases or enzymes, in particular in order to then test and/or detect the amplification products or nucleic-acid products. If RNA is intended to be tested and/or amplified, before the PCR is carried out, a cDNA is produced starting from the RNA, in particular using reverse transcriptase. The cDNA is used as a template for the subsequent PCR.

Preferably, during a PCR, a sample P is first denatured by the addition of heat in order to separate the strands of DNA or cDNA. Preferably, primers or nucleotides are then deposited on the individual separated strands of DNA or cDNA, and a desired DNA or cDNA sequence is replicated by means of polymerase and/or the missing strand is replaced by means of polymerase. This process is preferably repeated in a plurality of cycles until the desired quantity of the DNA or cDNA sequence is available.

For the PCR, marker primers are preferably used, i.e. primers which (additionally) produce a marker or a label L, in particular biotin, on the amplified analyte A or amplification product. This allows or facilitates detection. Preferably, the primers used are biotinylated and/or comprise or form in particular covalently bonded biotin as the label L.

The amplification products, target nucleic-acid sequences and/or other portions of the sample P produced in the one or more reaction cavities 109 can be conducted or fed to the connected sensor arrangement or sensor apparatus 113, in particular by means of the pump apparatus 112.

The sensor arrangement or sensor apparatus 113 is used in particular for detecting, particularly preferably qualitatively and/or quantitatively determining, the analyte A or analytes A of the sample P, in this case particularly preferably the target nucleic-acid sequences and/or target proteins as the analytes A. Alternatively or additionally, however, other values may also be collected or determined.

Preferably, the sensor arrangement or sensor apparatus 113 is provided with capture molecules M for bonding the analytes A. In particular, the sensor arrangement or sensor apparatus 113 is designed to electrochemically detect analytes A bonded to the capture molecules M.

The sensor arrangement or sensor apparatus 113 preferably comprises (precisely) one sensor array 113A comprising a plurality of sensor fields 113B and/or electrodes 113C, the sensor fields 113B and/or electrodes 113C each being in particular provided with capture molecules M.

Within the meaning of the present invention, capture molecules M are in particular nucleic-acid sequences, in particular DNA sequences and/or RNA sequences, and/or proteins, in particular antigens and/or antibodies. In particular, the capture molecules M are designed to bond and/or immobilise corresponding analytes A of the sample P.

Within the meaning of the present invention, capture molecules M are in particular applied to, fixed to and/or immobilised on a sensor array 113A, in particular the sensor fields 113B and/or electrodes 113C of the sensor array 113A, in a process known as spotting.

Preferably, the sensor array 113A, the sensor fields 113B and/or electrodes 113C are surface-treated or coated, in particular with thiols, in order to immobilise the capture molecules M, in particular in order to make it possible to bond the capture molecules M to the electrodes 113C.

In particular, the pump apparatus 112 comprises or forms a tube-like or bead-like raised portion, in particular by means of the film or cover 102, particularly preferably on the back of the cartridge 100, as shown schematically in Fig. 1.

The cartridge 100, the main body 101 and/or the fluid system 103 preferably comprise a plurality of channels 114 and/or valves 115, as shown in Fig. 2.

By means of the channels 114 and/or valves 115, the cavities 104 to 111, the pump apparatus 112 and/or the sensor arrangement or sensor apparatus 113 can be temporarily and/or permanently fluidically interconnected, in particular to form a fluidic circuit, and/or fluidically separated from one another, as required and/or optionally or selectively, in particular such that they are controlled by the analysis system 1 or the analysis device 200.

The cavities 104 to 111 are preferably each fluidically linked or interconnected by a plurality of channels 114. Particularly preferably, each cavity is linked or connected by at least two associated channels 114, in order to make it possible for fluid to fill, flow through and/or drain from the respective cavities as required.

The fluid transport or the fluid system 103 is preferably not based on capillary forces, or is not exclusively based on said forces, but in particular is essentially based on the effects of gravity and/or pumping forces and/or compressive forces and/or suction forces that arise, which are particularly preferably generated by the pump or pump apparatus 112. In this case, the flows of fluid or the fluid transport and the metering are controlled by accordingly opening and closing the valves 115 and/or by accordingly operating the pump or pump apparatus 112, in particular by means of a pump drive 202 of the analysis device 200.

Preferably, each of the cavities 104 to 110 has an inlet at the top and an outlet at the bottom in the operating position. Therefore, if required, only liquid from the respective cavities can be removed via the outlet.

In the operating position, the liquids from the respective cavities are preferably removed, in particular drawn out, via the outlet that is at the bottom in each case, it preferably being possible for gas or air to flow and/or be pumped into the respective cavities via the inlet that is in particular at the top. In particular, relevant vacuums in the cavities can thus be prevented or at least minimised when conveying the liquids.

In particular, the cavities, particularly preferably the storage cavity/cavities 108, the mixing cavity 107 and/or the receiving cavity 104, are each dimensioned and/or oriented in the normal operating position such that, when said cavities are filled with liquid, bubbles of gas or air that may potentially form rise upwards in the operating position, such that the liquid collects above the outlet without bubbles. However, other solutions are also possible here.

Preferably, in the normal operating position of the cartridge 100, the mixing cavity 107 and/or the cross-sectional area of the mixing cavity 107 enlarges towards the top and/or the cross-sectional area of the mixing cavity 107 diverges towards the top in the normal operating position of the cartridge 100. Owing to this type of construction, any bubbles can burst more easily on the (enlarged) liquid surface, and therefore foam formation and thus overflow of a fluid out of the mixing cavity 107 into adjacent channels and/or cavities is prevented or reduced.

The receiving cavity 104 preferably comprises a connection 104A for introducing the sample P. In particular, the sample P may for example be introduced into the receiving cavity 104 and/or cartridge 100 via the connection 104A by means of a pipette, syringe or other instrument.

The receiving cavity 104 preferably comprises an inlet 104B, an outlet 104C and an optional intermediate connection 104D, it preferably being possible for the sample P or a portion thereof to be removed and/or conveyed further via the outlet 104C and/or the optional intermediate connection 104D. Gas, air or another fluid can flow in and/or be pumped in via the inlet 104B, as already explained.

Preferably, the sample P or a portion thereof can be removed, optionally and/or depending on the assay to be carried out, via the outlet 104C or the optional intermediate connection 104D of the receiving cavity 104. In particular, a supernatant of the sample P, such as blood plasma or blood serum, can be conducted away or removed via the optional intermediate connection 104D, in particular for carrying out the protein assay.

Preferably, at least one valve 115 is assigned to each cavity, the pump apparatus 112 and/or the sensor arrangement or sensor apparatus 113 and/or is arranged upstream of the respective inlets and/or downstream of the respective outlets.

Preferably, the cavities 104 to 111 or sequences of cavities 104 to 111, through which fluid flows in series or in succession for example, can be selectively released and/or fluid can selectively flow therethrough by the assigned valves 115 being actuated, and/or said cavities can be fluidically connected to the fluid system 103, in particular a fluidic, preferably closed circuit of the fluid system 103, and/or to other cavities.

In particular, the valves 115 are formed by the main body 101 and the film or cover 102 and/or are formed therewith and/or are formed in another manner, for example by or having additional layers, depressions or the like.

Particularly preferably, one or more valves 115A are provided which are preferably tightly closed initially or at the factory or when delivered, particularly preferably in order to seal liquids or liquid reagents F, located in the storage cavities 108, and/or the fluid system 103 from the open receiving cavity 104 in a storage-stable manner. Preferably, an initially closed valve 115A is arranged upstream and downstream of each storage cavity 108. Said valves are preferably only opened, in particular automatically, when the cartridge 100 is actually being used and/or during or after (first) inserting the cartridge 100 into the analysis device 200 and/or for carrying out the assay.

A plurality of valves 115A, in particular three valves in this case, are preferably assigned to the receiving cavity 104, in particular if the intermediate connection 104D is provided in addition to the inlet 104B and the outlet 104C. Depending on the use, in addition to the valve 115A on the inlet 104B, then preferably only the valve 115A either at the outlet 104C or at the intermediate connection 104D is opened.

The valves 115A assigned to the receiving cavity 104 seal the fluid system 103 and/or the cartridge 100 in particular fluidically and/or in a gas-tight manner, preferably until the sample P is inserted and/or the receiving cavity 104 or the connection 104A of the receiving cavity 104 is closed.

As an alternative or in addition to the valves 115A (which are initially closed), one or more valves 115B are preferably provided which are not closed in a storage-stable manner and/or which are open initially or in an inoperative position, in an initial state or when the cartridge 100 is not inserted into the analysis device 200, and/or which can be closed by actuation. These valves 115B are used in particular to control the flows of fluid during the test.

The cartridge 100 is preferably designed as a microfluidic card and/or the fluid system 103 is preferably designed as a microfluidic system. In the present invention, the term "microfluidic" is preferably understood to mean that the respective volumes of individual cavities, some of the cavities or all of the cavities 104 to 111 and/or channels 114 are, separately or cumulatively, less than 5 ml or 2 ml, particularly preferably less than 1 ml or 800 µl, in particular less than 600 µl or 300 µl, more particularly preferably less than 200 µl or 100 µl.

Particularly preferably, a sample P having a maximum volume of 5 ml, 2 ml or 1 ml can be introduced into the cartridge 100 and/or the fluid system 103, in particular the receiving cavity 104.

Reagents and liquids which are preferably introduced or provided before the test in liquid form as liquids or liquid reagents F and/or in dry form as dry reagents S are required for testing the sample P, as shown in the schematic view according to Fig. 2.

Furthermore, other liquids F, in particular in the form of a wash buffer, solvent for dry reagents S and/or a substrate SU, for example in order to form detection molecules D and/or a redox system, are also preferably required for the testing, the detection process and/or for other purposes, and are in particular provided in the cartridge 100, i.e. are likewise introduced before use, in particular before delivery. At some points in the following, a distinction is not made between liquid reagents and other liquids, and therefore the respective explanations are accordingly also mutually applicable.

The analysis system 1 or the cartridge 100 preferably contains all the reagents and liquids required for pretreating the sample P and/or for carrying out the test or assay, in particular for carrying out one or more amplification reactions or PCRs, and therefore, particularly preferably, it is only necessary to receive the optionally pretreated sample P.

The cartridge 100 or the fluid system 103 preferably comprises a bypass 114A that can optionally be used, in order for it to be possible, if necessary, to conduct or convey the sample P or components thereof past the reaction cavities 109 and/or, by bypassing the optional intermediate temperature-control cavity 110, also directly to the sensor arrangement or sensor apparatus 113.

Preferably, the bypass 114A is used when carrying out the protein assay, in particular in order to feed the sample P or a portion thereof directly from the mixing cavity 107 to the sensor arrangement or sensor apparatus 113, and/or to conduct said sample or portion past the reaction cavities 109 and/or the intermediate temperature-control cavity 110.

The cartridge 100 or the fluid system 103 or the channels 114 preferably comprise sensor portions 116 or other apparatuses for detecting liquid fronts and/or flows of fluid.

As can be seen in particular in Fig. 2, the sensor portions 116 are designed as preferably elongate cavities, the longitudinal extension of the sensor portions 116 preferably extending at least substantially vertically, and/or in parallel with gravity G in the normal operating position of the cartridge 100.

More particularly preferably, the sensor portions 116 are arranged such that fluid flows therethrough at least substantially vertically, in particular from the bottom to the top, in the normal operating position of the cartridge 100. Advantageously, the effect of gravity G on the detection of liquid fronts or flows of fluid is thus reduced. In particular, a liquid front or flow of fluid extending transversely to the longitudinal extension of the respective sensor portions 116 is generated and bubble formation and/or foaming of the fluid in the sensor portions 116 is counteracted.

It is noted that various components, such as the channels 114, the valves 115, in particular the valves 115A that are initially closed and the valves 115B that are initially open, and the sensor portions 116 in Fig. 2 are, for reasons of clarity, only labelled in some cases, but the same symbols are used in Fig. 2 for each of these components.

The collection cavity 111 is preferably used for receiving excess or used reagents and liquids and volumes or portions of the sample, and/or for providing gas or air in order to empty individual cavities and/or channels. In the initial state, the collection cavity 111 is preferably filled solely with gas, in particular air.

In particular, the collection cavity 111 can optionally be connected to individual cavities and channels or other apparatuses fluidically and/or so as to form a fluidic circuit, in order to remove reagents and liquids from said cavities, channels or other apparatuses and/or to replace said reagents and liquids with gas or air in particular from the collection cavity 111. The collection cavity 111 is preferably given appropriate (large) dimensions.

Once the sample P has been introduced into the receiving cavity 104 and the connection 104A has been closed, the cartridge 100 can be inserted into and/or received in the proposed analysis device 200 in order to test the sample P, as shown in Fig. 1. Alternatively, the sample P could also be fed in later.

Fig. 1 shows the analysis system 1 in a ready-to-use state for carrying out a test or assay on the sample P received in the cartridge 100. In this state, the cartridge 100 is therefore linked to, received by and/or inserted into the analysis device 200.

In the following, some features and aspects of the analysis device 200 are first explained in greater detail, in particular on the basis of Fig. 1. The features and aspects relating to said device are preferably also directly features and aspects of the proposed analysis system 1, in particular even without any further explicit explanation.

The analysis system 1 or analysis device 200 preferably comprises an in particular slot-like mount or receptacle 201 for preferably vertically mounting and/or receiving the cartridge 100.

Preferably, the cartridge 100 is fluidically, in particular hydraulically, separated or isolated from the analysis device 200. In particular, the cartridge 100 forms a preferably independent and in particular closed or sealed fluidic or hydraulic system 103 for the sample P and the reagents and other liquids. In this way, the analysis device 200 does not come into direct contact with the sample P and can in particular be reused for another test without being disinfected and/or cleaned first.

It is however provided that the analysis device 200 is or can be connected or coupled mechanically, electrically, thermally and/or pneumatically to the cartridge 100, in particular on one of the flat sides of the cartridge 100 and/or laterally. In particular, after receiving the cartridge 100, the analysis device 200 mechanically, thermally and/or pneumatically acts on the cartridge 100 on at least one of the flat sides of the cartridge 100 and/or laterally.

In particular, the analysis device 200 is designed to have a mechanical effect, in particular for actuating the pump apparatus 112 and/or the valves 115, and/or to have a thermal effect, in particular for temperature-controlling the reaction cavity/cavities 109 and/or the intermediate temperature-control cavity 110.

In addition, the analysis device 200 can preferably be pneumatically connected to the cartridge 100, in particular in order to actuate individual apparatuses, and/or can be electrically connected to the cartridge 100, in particular in order to collect and/or transmit measured values, for example from the sensor apparatus 113 and/or sensor portions 116.

The analysis system 1 or analysis device 200 preferably comprises a pump drive 202, the pump drive 202 in particular being designed for mechanically actuating the pump apparatus 112.

Preferably, a head of the pump drive 202 can be rotated in order to actuate and/or rotationally axially depress the preferably bead-like raised portion of the pump apparatus 112. Particularly preferably, the pump drive 202 and pump apparatus 112 together form a pump, in particular in the manner of a hose pump or peristaltic pump and/or a metering pump, for the fluid system 103 and/or the cartridge 100.

Particularly preferably, the pump is constructed as described in DE 10 2011 015 184 B4. However, other structural solutions are also possible.

Preferably, the capacity and/or discharge rate of the pump can be controlled and/or the conveying direction of the pump, pump drive 202 and/or of fluids in the cartridge 100 can be switched. Preferably, fluid can thus be pumped forwards or backwards as desired, as explained in greater detail in the following.

The analysis system 1 or analysis device 200 preferably comprises a connection apparatus 203 for in particular electrically and/or thermally connecting the cartridge 100, in particular the sensor arrangement or sensor apparatus 113.

As shown in Fig. 1, the connection apparatus 203 preferably comprises a plurality of electrical contact elements 203A, the cartridge 100, in particular the sensor arrangement or sensor apparatus 113, preferably being electrically connected or connectable to the analysis device 200 by the contact elements 203A. The contact elements 203A are preferably contact springs; however, they may also be springloaded connection pins or the like.

The analysis system 1 or analysis device 200 preferably comprises one or more temperature-control apparatuses 204 for temperature-controlling the cartridge 100 and/or having a thermal effect on the cartridge, in particular for heating and/or cooling, the temperature-control apparatus(es) 204 (each) preferably comprising or being formed by a heating resistor or a Peltier element.

Individual temperature-control apparatuses 204, some of these apparatuses or all of these apparatuses can preferably be positioned against the cartridge 100, the main body 101, the cover 102, the sensor arrangement, sensor apparatus 113 and/or individual cavities and/or can be thermally coupled thereto and/or can be integrated therein and/or in particular can be operated or controlled electrically by the analysis device 200. In the example shown, in particular the temperature-control apparatuses 204A, 204B and/or 204C are provided.

Preferably, the temperature-control apparatus 204A, referred to in the following as the reaction temperature-control apparatus 204A, is assigned to the reaction cavity 109 or to a plurality of reaction cavities 109, in particular in order for it to be possible to carry out one or more amplification reactions therein.

When the cartridge 100 is inserted, the reaction temperature-control apparatus 204A preferably abuts the cartridge 100 in the region of the reaction cavity/cavities 109, and therefore a fluid located in said cartridge, in particular the sample P or portions thereof, can be heated and/or cooled.

The reaction cavities 109 are preferably temperature-controlled simultaneously and/or uniformly, in particular by means of one common reaction temperature-control apparatus 204A or two reaction temperature-control apparatuses 204A.

Alternatively, each reaction cavity 109 can be temperature-controlled independently and/or individually.

More particularly preferably, the reaction cavity/cavities 109 can be temperature-controlled from two different sides and/or by means of two or the reaction temperature-control apparatuses 204A that are preferably arranged on opposite sides.

The temperature-control apparatus 204B, referred to in the following as the intermediate temperature-control apparatus 204B, is preferably assigned to the intermediate temperature-control cavity 110 and/or is designed to (actively) temperature-control or heat the intermediate temperature-control cavity 110 or a fluid located therein, in particular the analytes A, amplification products and/or target nucleic-acid sequences, preferably to a preheat temperature.

The intermediate temperature-control cavity 110 and/or intermediate temperature-control apparatus 204B is preferably arranged upstream of or (immediately) before the sensor arrangement or sensor apparatus 113, in particular in order for it to be possible to temperature-control or preheat, in a desired manner, fluids to be fed to the sensor arrangement or sensor apparatus 113, in particular analytes A, amplification products and/or target nucleic-acid sequences, particularly preferably immediately before said fluids are fed.

Particularly preferably, the intermediate temperature-control cavity 110 or intermediate temperature-control apparatus 204B is designed or intended to denature the sample P, analytes A, the amplification products and/or target nucleic-acid sequences produced, and/or to divide any double-stranded analytes A, amplification products and/or target nucleic-acid sequences into single strands and/or to counteract premature bonding or hybridising of the amplification products and/or target nucleic-acid sequences, in particular by the addition of heat.

Preferably, the analysis system 1, analysis device 200 and/or the cartridge 100 and/or one or each temperature-control apparatus 204 comprise a temperature detector and/or temperature sensor (not shown), in particular in order to make it possible to control and/or feedback control temperature.

One or more temperature sensors may for example be assigned to the sensor portions 116 and/or to individual channel portions or cavities, i.e. may be thermally coupled thereto.

The temperature-control apparatus 204C, referred to in the following as the sensor temperature-control apparatus 204C, is in particular assigned to the sensor apparatus 113 and/or is designed to (actively) temperature-control or heat fluids located in or on the sensor arrangement or sensor apparatus 113, in particular analytes A or target proteins or target nucleic-acid sequences, in a desired manner, in particular in order to bond and/or to (then) dissolve or denature said fluids.

The sensor temperature-control apparatus 204C is preferably planar and/or has a contact surface which is preferably rectangular and/or corresponds to the dimensions of the sensor arrangement or sensor apparatus 113, the contact surface allowing for heat transfer between the sensor temperature-control apparatus 204C and the sensor apparatus 113.

Preferably, the analysis device 200 comprises the sensor temperature-control apparatus 204C. However, other structural solutions are also possible in which the sensor temperature-control apparatus 204C is integrated in the cartridge 100, in particular the sensor arrangement or sensor apparatus 113.

Particularly preferably, the connection apparatus 203 comprises the sensor temperature-control apparatus 204C, and/or the connection apparatus 203 together with the sensor temperature-control apparatus 204C can be linked to, in particular pressed against, the cartridge 100, in particular the sensor arrangement or sensor apparatus 113.

More particularly preferably, the connection apparatus 203 and the sensor temperature-control apparatus 204C (together) can be moved counter to, towards and/or relative to the cartridge 100, in particular the sensor arrangement or sensor apparatus 113, and/or can be positioned against or abutted on said cartridge, preferably in order to both electrically and thermally couple the analysis device 200 to the cartridge 100, in particular the sensor arrangement or sensor apparatus 113 or the support 113D thereof.

Preferably, the sensor temperature-control apparatus 204C is arranged centrally on the connection apparatus 203 or a support thereof and/or is arranged between the contact elements 203A.

In particular, the contact elements 203A are arranged in an edge region of the connection apparatus 203 or a support thereof or are arranged around the sensor temperature-control apparatus 204C, preferably such that the connection apparatus 203 is connected or connectable to the sensor apparatus 113 thermally in the centre and electrically on the outside or in the edge region. However, other solutions are also possible here.

The analysis system 1 or analysis device 200 preferably comprises one or more actuators 205 for actuating the valves 115. Particularly preferably, different (types or groups of) actuators 205A and 205B are provided which are assigned to the different (types or groups of) valves 115A and 115B for actuating each of said valves, respectively.

The analysis system 1 or analysis device 200 preferably comprises one or more sensors 206. In particular, sensors 206A are assigned to the sensor portions 116 and/or are designed or intended to detect liquid fronts and/or flows of fluid in the fluid system 103.

Particularly preferably, the sensors 206A are designed to measure or detect, in particular in a contact-free manner, for example optically and/or capacitively, a liquid front, flow of fluid and/or the presence, the speed, the mass flow rate/volume flow rate, the temperature and/or another value of a fluid in a channel and/or a cavity, in particular in a respectively assigned sensor portion 116, which is in particular formed by a planar and/or widened channel portion of the fluid system 103.

Particularly preferably, the sensor portions 116 are each oriented and/or incorporated in the fluid system 103 and/or fluid flows against or through the sensor portions 116 such that, in the operating position of the cartridge 100, fluid flows through the sensor portions 116 in the vertical direction and/or from the bottom to the top, or vice versa, in particular in order to make it possible or easier to accurately detect liquid, as already explained at the outset.

Alternatively or additionally, the analysis device 200 preferably comprises (other or additional) sensors 206B for detecting the ambient temperature, internal temperature, atmospheric humidity, position, and/or alignment, for example by means of a GPS sensor, and/or the orientation and/or inclination of the analysis device 200 and/or the cartridge 100.

Particularly preferably, the analysis device 200 comprises a sensor 206B for detecting the horizontal and/or vertical orientation of the cartridge 100 and/or analysis device 200, the sensor 206B preferably being designed as a tilt sensor or inclinometer. However, other solutions are also possible here, in particular those in which the analysis device 200 comprises a spirit level or level indicator in order to display the horizontal and/or vertical orientation of the cartridge 100 and/or analysis device 200.

The analysis system 1 or analysis device 200 preferably comprises a control apparatus 207, in particular comprising an internal clock or time base for controlling the sequence of a test or assay and/or for collecting, evaluating and/or outputting or providing measured values in particular from the sensor apparatus 113, and/or from test results and/or other data or values.

The control apparatus 207 preferably controls or feedback controls the pump drive 202, the temperature-control apparatuses 204 and/or actuators 205, in particular taking into account or depending on the desired test and/or measured values from the sensor arrangement or sensor apparatus 113 and/or sensors 206.

The flows of fluid are controlled in particular by accordingly activating the pump or pump apparatus 112 and actuating the valves 115.

Particularly preferably, the pump drive 202 comprises a servomotor, stepper motor, or a drive calibrated in another way or a drive having a rotational speed and/or number of (partial) revolutions that can be controlled or feedback controlled, such that desired metering can be achieved, at least in principle, by means of appropriate activation.

Additionally or alternatively, the sensors 206A are used to detect liquid fronts or flows of fluid, in particular in cooperation with the assigned sensor portions 116, in order to achieve the desired fluidic sequence and/or the desired metering by accordingly controlling the pump or pump apparatus 112 and accordingly activating the valves 115.

Optionally, the analysis system 1 or analysis device 200 comprises an input apparatus 208, such as a keyboard, a touch screen or the like, and/or a display apparatus 209, such as a screen.

The analysis system 1 or analysis device 200 preferably comprises at least one interface 210, for example for controlling, for communicating and/or for outputting measured data or test results and/or for linking to other devices, such as a printer, an external power supply or the like. This may in particular be a wired or wireless interface 210.

The analysis system 1 or analysis device 200 preferably comprises a power supply 211 for providing electrical power, preferably a battery or an accumulator, which is in particular integrated and/or externally connected or connectable.

Preferably, an integrated accumulator is provided as a power supply 211 and is (re)charged by an external charging device (not shown) via a connection 211A and/or is interchangeable.

The analysis system 1 or analysis device 200 preferably comprises a housing 212, all the components and/or some or all of the apparatuses preferably being integrated in the housing 212. Particularly preferably, the cartridge 100 can be inserted or slid into the housing 212 or the mount 201, and/or can be received by the analysis device 200 or the mount 201, through an opening 213 which can in particular be closed, such as a slot or the like.

The analysis system 1 or analysis device 200 is preferably portable or mobile. Preferably, the analysis device 200 weighs less than 25 kg or 20 kg, particularly preferably less than 15 kg or 10 kg, in particular less than 9 kg or 6 kg.

As already explained, the analysis device 200 can preferably be pneumatically linked to the cartridge 100, in particular to the sensor arrangement and/or to the pump apparatus 112.

Particularly preferably, the analysis device 200 is designed to supply the cartridge 100, in particular the sensor arrangement and/or the pump apparatus 112, with a working medium, in particular gas or air.

Preferably, the working medium can be compressed and/or pressurised in the analysis device 200 or by means of the analysis device 200.

Preferably, the analysis device 200 comprises a pressurised gas supply 214, in particular a pressure generator and/or compressor, preferably in order to compress, condense and/or pressurise the working medium.

The pressurised gas supply 214 is preferably integrated in the analysis device 200 or the housing 212 and/or can be controlled or feedback controlled by means of the control apparatus 207.

Preferably, the pressurised gas supply 214 is electrically operated or can be operated by electrical power. In particular, the pressurised gas supply 214 can be supplied with electrical power by means of the power supply 211.

Preferably, air can be drawn in, in particular from the surroundings, as the working medium by means of the analysis device 200 or pressurised gas supply 214. In particular, the analysis device 200 or pressurised gas supply 214 is designed to use the surroundings as a reservoir for the working medium or the air. However, other solutions are also possible here, in particular those in which the analysis device 200 or pressurised gas supply 214 comprises a preferably closed or delimited reservoir, such as a tank or container, comprising the working medium, and/or is connected or connectable thereto.

The analysis device 200 or pressurised gas supply 214 preferably comprises a connection element 214A, in particular in order to pneumatically connect the analysis device 200 or pressurised gas supply 214 to the cartridge 100.

Preferably, the analysis device 200, in particular the housing 212, comprises a support apparatus 215 for providing support at the base. In particular, the support apparatus 215 is designed to absorb and/or compensate for forces, movements and/or vibrations and/or to dissipate said forces, movements and/or vibrations at the base.

Preferably, the support apparatus 215 comprises at least one spring and/or at least one damper, and/or the support apparatus 215 is formed by at least one spring and/or one damper and/or a spring/damper system. However, other solutions are also possible here.

Particularly preferably, the support apparatus 215 is variable and/or (height) adjustable. In particular, the analysis device 200 can be oriented, in particular horizontally or vertically, by means of the support apparatus 215, in particular such that the cartridge 100 is oriented at least substantially vertically in the analysis device 200 for the test, and/or that the main plane of extension H of the cartridge 100 extends at least substantially vertically.

In the embodiment shown, the analysis device 200 or the support apparatus 215 comprises a plurality of support elements or feet 215A, which are in particular variable and/or (height) adjustable, it preferably being possible for the horizontal and/or vertical orientation and/or the inclination of the analysis device 200, and therefore of the cartridge 100 that is received or to be received in the analysis device 200, to be set or adapted by moving, in particular rotating, the support elements 215A. However, other solutions are also possible here.

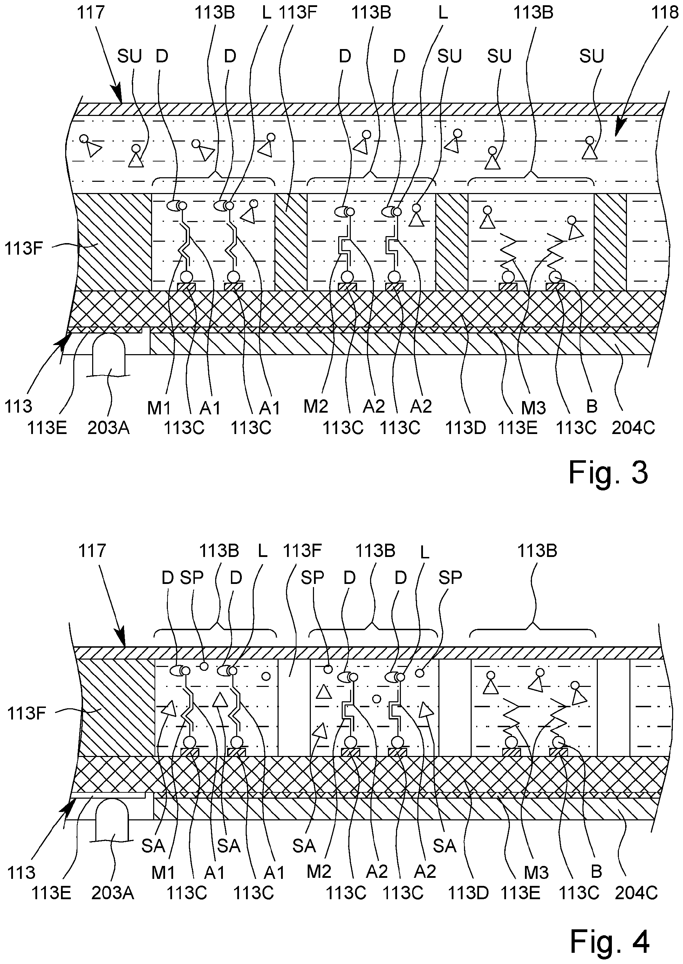

In the following, further details are given on a preferred construction and the preferred mode of operation of the sensor arrangement with reference to Fig. 3 and Fig. 4.

The sensor arrangement preferably comprises the sensor apparatus 113, a sensor cover 117 for the sensor apparatus 113 that is preferably flexible at least in part, (precisely) one sensor compartment 118, an inlet 119 into the sensor compartment 118 and/or an outlet 120 out of the sensor compartment 118.

The sensor arrangement, in particular the sensor apparatus 113, is preferably designed for electrochemically measuring or detecting analytes A of the sample P. Preferably, detection or measuring of the analytes A of the sample P takes place or is performed exclusively in the sensor apparatus 113 or the (precisely) one sensor compartment 118.

In particular, the sensor arrangement or sensor apparatus 113 is designed to detect, to identify and/or to determine (identical or different) analytes A bonded to capture molecules M or products derived therefrom, in particular amplification products of the analyte A or different analytes A.

The sensor arrangement is preferably designed as a multiple-part module, the sensor apparatus 113 and the sensor cover 117 preferably each forming a component of the sensor arrangement or module. In particular, the components of the sensor arrangement are directly interconnected.

Preferably, the sensor arrangement has a layered, in particular compact, construction, the sensor apparatus 113 preferably forming a base of the sensor arrangement and the sensor cover 117 being directly connected to the sensor apparatus 113, at least at the edge, and/or resting thereon.

The sensor apparatus 113 and the sensor cover 117 define or delimit the sensor compartment 118, preferably on the flat sides. In particular, the sensor compartment 118 is formed or arranged between the sensor apparatus 113 and the sensor cover 117.

The sensor compartment 118 preferably has, in particular when the sensor cover 117 is not actuated or has been moved away, a volume of greater than 0.1 µl or 0.2 µl, particularly preferably greater than 0.5 µl or 1 µl, in particular greater than 2 µl, and/or less than 10 µl or 8 µl, particularly preferably less than 6 µl or 3 µl.

The sensor arrangement, in particular the sensor apparatus 113 and the sensor cover 117, is/are preferably planar, flat and/or plate-shaped. Preferably, the surface area of a flat side of the sensor apparatus 113 and/or sensor cover 117 is less than 400 mm² or 300 mm², particularly preferably less than 250 mm² or 150 mm², in particular less than 100 mm² or 50 mm², and/or greater than 0.01 mm² or 0.25 mm², particularly preferably greater than 1 mm² or 4 mm².

The sensor apparatus 113 preferably has a front side or measuring side and a rear side or connection side, the measuring side and the connection side each preferably forming one flat side of the in particular flat, planar and/or plate-shaped sensor apparatus 113.

The measuring side is preferably the side of the sensor apparatus 113 facing the fluid or the sample P or the analytes A or the sensor compartment 118.

The connection side is preferably opposite the measuring side and/or is the side of the sensor apparatus 113 that faces away from the fluid or the sample P or the analytes A or the sensor compartment 118.

The sensor apparatus 113 preferably comprises (precisely) one sensor array 113A on the measuring side, having a plurality of sensor cavities and/or sensor fields 113B, the sensor fields 113B preferably being round, in particular circular, in a plan view of the sensor array 113A and/or being arranged so as to be electrically isolated from one another and/or directly next to one another.

Fig. 3 and Fig. 4 are each schematic sections through the sensor arrangement during different method steps.

Fig. 3 is a schematic section through the sensor arrangement with the sensor cover 117 moved away and/or immediately before the measurement and/or during pretreatment. Fig. 4 is a schematic section through the sensor arrangement with the sensor cover 117 lowered and/or during the measurement of the bonded analytes A.

Preferably, the sensor arrangement or sensor apparatus 113 or the sensor array 113A comprises more than 10 or 20, particularly preferably more than 50 or 80, in particular more than 100 or 120 and/or less than 1000 or 800 sensor fields 113B.

Preferably, the sensor fields 113B are separated or spaced apart from one another, in particular by less than 100 µm or 10 µm and/or more than 10 nm or 100 nm. Particularly preferably, all the sensor fields 113B are arranged on a surface area of less than 100 mm² and/or greater than 1 mm² and/or the sensor array 113A has a surface area of less than 100 mm² and/or greater than 1 mm².

Preferably, the sensor apparatus 113 comprises barriers or partitions between each of the sensor fields 113B, which are preferably formed by an in particular hydrophobic layer 113F having corresponding recesses for the sensor fields 113B. However, other structural solutions are also possible.

Preferably, the sensor arrangement or sensor apparatus 113 or the sensor array 113A comprises a plurality of electrodes 113C. Particularly preferably, at least two electrodes 113C are arranged in each sensor field 113B. In particular, at least or precisely two electrodes 113C corresponding to one another form one or each sensor field 113B.

The electrodes 113C are preferably made of metal so as to be electrically conductive, in particular at least the surface thereof is made of noble metal, such as platinum or gold, and/or said electrodes are coated, in particular with thiols.

Preferably, the electrodes 113C are finger-like and/or engage in one another. However, other structural solutions or arrangements are also possible.

The sensor apparatus 113 preferably comprises a support 113D, in particular a chip, the electrodes 113C preferably being arranged on the support 113D and/or being integrated in the support 113D.

The sensor apparatus 113, in particular the support 113D, preferably comprises a plurality of electrical contacts or contact surfaces 113E, the contacts 113E preferably being arranged on the connection side and/or forming the connection side, as shown in Fig. 3 and Fig. 4.

Preferably, the sensor apparatus 113 can be electrically contacted on the connection side and/or by means of the contacts 113E and/or can be electrically connected to the analysis device 200. In particular, an electrical connection can be established between the cartridge 100, in particular the sensor apparatus 113, and the analysis device 200, in particular the control apparatus 207, by electrically connecting the contacts 113E to the contact elements 203A of the connection apparatus 203.

Preferably, the contacts 113E are arranged laterally, in the edge region and/or in a plan view or projection around the electrodes 113C and/or the sensor array 113A, and/or the contacts 113E extend as far as the edge region of the sensor apparatus 113, in particular such that the sensor apparatus 113 can be electrically contacted, preferably by means of the connection apparatus 203 or the contact elements 203A, laterally, in the edge region and/or around the sensor temperature-control apparatus 204C, which can preferably be positioned centrally or in the middle on the support 113D.

As already explained, the sensor compartment 118 is preferably arranged between the sensor apparatus 113 and the sensor cover 117, the measurement side and/or the sensor array 113A of the sensor apparatus 113 preferably defining or delimiting the sensor compartment 118.

Preferably, all the sensor fields 113B and/or all the electrodes 113C are fluidically interconnected by the (common) sensor compartment 118, in particular such that all the sensor fields 113B and/or electrodes 113C can come into contact with a fluid, the sample P and/or the analytes A via the (common) sensor compartment 118.

The sensor cover 117 can preferably be actuated and/or can be moved relative to the sensor apparatus 113. In particular, the sensor cover 117 can be lowered onto the sensor apparatus 113, in particular the sensor array 113A and/or the layer 113F, preferably such that the sensor fields 113B are closed and/or fluidically separated from one another. Particularly preferably, the sensor cover 117 can be actuated pneumatically and/or by means of the pressurised gas supply 214. However, other solutions are also possible here.

In particular, the fluid can be displaced out of the sensor compartment 118 by means of the sensor cover 117, and/or by lowering the sensor cover 117 onto the sensor apparatus 113.

The sensor cover 117 is therefore designed to seal and/or fluidically separate the individual sensor fields 113B from one another for the actual measurement, preferably such that fluid cannot be exchanged between the sensor fields 113B, at least when the measurement is being taken.

At least when the sensor cover 117 is moved away, the sensor apparatus 113 or the sensor compartment 118 is fluidically linked to the fluid system 103, in particular to the reaction cavity/cavities 109, preferably by the inlet 119 and the outlet 120, in particular such that fluids, in particular the (pretreated) sample P or portions thereof or the analytes A and/or reagents, can be admitted to the measurement side of the sensor apparatus 113 or sensor array 113A.

The sensor compartment 118 can thus be loaded with fluids and/or said fluids can flow therethrough, at least when the sensor cover 117 is raised or moved away from the sensor apparatus 113 or the sensor array 113A.

Preferably, fluid can flow through the sensor compartment 118 by means of the inlet 119 and the outlet 120. In particular, a fluid can flow into the sensor compartment 118 via the inlet 119 and can flow out of the sensor compartment 118 via the outlet 120; however, the flow direction or conveying direction can also be reversed. In particular, the inlet 119 can be designed or used as the outlet, at least temporarily, and the outlet 120 can be designed or used as the inlet, at least temporarily.

The inlet 119 and/or the outlet 120 is/are preferably formed by cut-outs, holes, openings, channels or the like in the main body 101, the sensor cover 117 and/or the sensor apparatus 113.

Preferably, the inlet 119 is at the bottom in the normal operating position of the cartridge 100 and the outlet 120 is at the top in the normal operating position of the cartridge 100, in particular such that fluid can flow through the sensor arrangement or the sensor compartment 118 vertically, and/or from the bottom to the top, or vice versa. This in particular ensures that the sensor arrangement or the sensor compartment 118 is completely filled and/or fluid flows through the entirety thereof, and/or it is ensured that no bubbles, remnants, sample residues or the like remain in the sensor arrangement or the sensor compartment 118.

The sensor apparatus 113 preferably comprises a plurality of in particular different capture molecules M for bonding the analytes A, different capture molecules M preferably being arranged and/or immobilised in or on different sensor fields 113B and/or being assigned to different sensor fields 113B.

Fig. 3 and Fig. 4 show, by way of example, three different sensor fields 113B, each sensor field 113B comprising different capture molecules M1, M2 or M3, respectively. In the example, different analytes A1 and A2 have already bonded to the corresponding capture molecules M1 and M2.

Particularly preferably, the sensor fields 113B or electrodes 113C are provided with the capture molecules M, in particular already when the cartridge is delivered or at the factory, and/or the capture molecules M are immobilised or fixed in or on the sensor fields 113B or electrodes 113C, in particular as already when the cartridge is delivered or at the factory.

As already explained at the outset, the capture molecules M are preferably capture proteins, in particular capture antigens and/or capture antibodies, and capture nucleic-acid sequences, in particular capture DNA sequences, oligonucleotides or fragments of PCR products.

Preferably, the capture molecules M are fixed to the sensor apparatus 113 or the sensor array 113A or electrodes 113C by a bond B, in particular a thiol bond, and/or what is known as a spacer, in particular a C6 spacer. The formation of structures that disrupt hybridisation, e.g. hairpin structures, can be prevented by the preferred bonding of the capture molecules M by the bond B.

Different capture proteins and/or different capture nucleic-acid sequences are preferably provided for the different sensor fields 113B and/or the different electrode pairs and/or electrodes 113C, in order to specifically bond different analytes A, in particular different target proteins and/or target nucleic-acid sequences, in the sensor fields 113B.

Particularly preferably, the sensor apparatus 113 or sensor array 113A allows the analytes A bonded in each sensor field 113B to be qualitatively or quantitatively determined.

Optionally, the sensor apparatus 113 comprises capture molecules M having different hybridisation temperatures, preferably in order to bond the analytes A, in particular target nucleic-acid sequences, to the corresponding capture molecules M at different hybridisation temperatures.

The hybridisation temperature is preferably the (average) temperature at which an (amplified) analyte A or a target nucleic-acid sequence or a target protein is bonded to a corresponding capture molecule M or a corresponding capture nucleic-acid sequence or a corresponding capture protein.

The optimal hybridisation temperature is preferably the temperature at which the number of analytes A bonded to corresponding capture molecules M is maximised and/or the number of analytes A bonded to one another is minimised.

Preferably, the (optimal) hybridisation temperature varies for different analytes A, in particular target nucleic-acid sequences.

Preferably, the temperature of the sensor apparatus 113, in particular of the electrodes 113C, the support 113D, the sensor compartment 118 and/or the sensor cover 117, can be controlled or set, at least indirectly, preferably by means of the analysis device 200, in particular the sensor temperature-control apparatus 204C, as already explained.

Preferably, the sensor temperature-control apparatus 204C is used to temperature-control the sensor compartment 118, in this case by being in contact with the connection side, in particular such that the desired or required or optimal denaturing temperature and/or hybridisation temperature is set on the measuring side and/or in the sensor compartment 118.

Preferably, in the operating state, the sensor temperature-control apparatus 204C rests on or contacts the support 113D in a planar manner and/or centrally and/or so as to be opposite the sensor array 113A and/or rests on or contacts one or more contacts 113E at least in part. This makes it possible to particularly rapidly and efficiently temperature-control the sensor compartment 118 and/or the capture molecules M and analytes A.

The sensor apparatus 113, in particular the support 113D, preferably comprises at least one, preferably a plurality of, electronic or integrated circuits, the circuits in particular being designed to detect electrical currents or voltages that are preferably generated at the sensor fields 113B in accordance with the redox cycling principle. Particularly preferably, the measurement signals from the different sensor fields 113B are separately collected or measured by the sensor apparatus 113 and/or the circuits.

Particularly preferably, the sensor apparatus 113 or the integrated circuits directly convert the measurement signals into digital signals or data, which can in particular be read out by or using the analysis device 200.

Particularly preferably, the sensor apparatus 113 or the support 113D is constructed as described in EP 1 636 599 B1.

In the following, a preferred sequence of a test or analysis using the proposed analysis system 1 and/or analysis device 200 and/or the proposed cartridge 100 and/or in accordance with the proposed method is explained in greater detail by way of example.

The analysis system 1, the cartridge 100 and/or the analysis device 200 is preferably designed to carry out the proposed method.

In the proposed method, a nucleic-acid assay is preferably carried out in order to detect or identify a target nucleic-acid sequence, in particular a target DNA sequence and/or target RNA sequence. Particularly preferably, target nucleic-acid sequences are bonded to corresponding capture molecules M, in particular capture nucleic-acid sequences, in the form of analytes A of the sample P.

Additionally or alternatively, a protein assay is carried out in order to detect or identify a target protein, in particular a target antigen and/or target antibody. In particular, target proteins are bonded to corresponding capture molecules M, in particular capture proteins, in the form of analytes A of the sample P.

During the nucleic-acid assay, at least one analyte A of the sample P is preferably amplified or copied, in particular by means of PCR. A method step of this type is preferably omitted when carrying out the protein assay.

Unless specified more precisely, the method steps described in the following are in principle preferably provided in both the nucleic-acid assay and the protein assay. In particular, the bonded analytes A or the amplification products thereof are electrochemically identified or detected both in the nucleic-acid assay and the protein assay.

The method may be used in particular in the field of medicine, in particular veterinary medicine, for example in order to detect or identify diseases and/or pathogens in a sample P.

At the start of the proposed method, a sample P having at least one analyte A, preferably a fluid or a liquid from the human or animal body, in particular blood, saliva or urine, is preferably first introduced into the receiving cavity 104 via the connection 104A, it being possible for the sample P to be pretreated, in particular filtered.

Once the sample P has been received, the receiving cavity 104 and/or the connection 104A thereof is fluidically closed, in particular in a liquid-tight and/or gas-tight manner.

Preferably, the cartridge 100 together with the sample P is then linked to the analysis device 200, in particular is inserted or slid at least in part into the analysis device 200 or the mount 201 or opening 213, particularly preferably from the top.

Particularly preferably, the cartridge 100 is received at least in part, at least substantially vertically, by the analysis device 200.

Preferably, the in particular vertical and/or horizontal orientation of the cartridge 100 and/or the analysis device 200 is measured, in particular electronically and/or by means of the sensor 206B, preferably before the test starts.

In particular, the in particular vertical and/or horizontal orientation of the cartridge 100 or the analysis device 200 is measured, in particular by means of the sensor 206B, immediately after the analysis device 200 is switched on and/or after the cartridge 100 is received. In particular, it is measured or established whether the main plane of extension H of the cartridge 100 extends vertically in the analysis device 200 and/or whether the analysis device 200 is oriented horizontally and/or positioned so as to be flat and/or is not tilted and/or not inclined.

Preferably, the measured orientation of the cartridge 100 and/or the analysis device 200 is displayed to a user, preferably by the display apparatus 209.

Preferably, the test is blocked or prevented, in particular the test is blocked or prevented from starting, particularly preferably electronically, if the orientation of the cartridge 100 is inclined or not vertical and/or if the orientation of the analysis device 200 is tilted or not horizontal. More particularly preferably, the sample P can only be tested when the cartridge 100 is at least essentially oriented vertically and/or when the analysis device 200 is at least essentially oriented horizontally.

If the cartridge 100 or the analysis device 200 is oriented so as to be inclined or tilted and/or is not oriented as desired, the orientation of the analysis device 200 and thus of the cartridge 100 is adapted, preferably by adjusting the support apparatus 215, in particular the support elements 215A.

In particular, the analysis device 200 can be oriented by vertically adjusting the support apparatus 215 or the support elements 215A such that the main plane of extension H of the cartridge 100 extends vertically in the analysis device 200, in particular irrespective of any unevenness in the floor or surface underneath.

Preferably, it is displayed, in particular by means of the display apparatus 209, when the correct or vertical orientation is set. The testing of the sample P can then start.

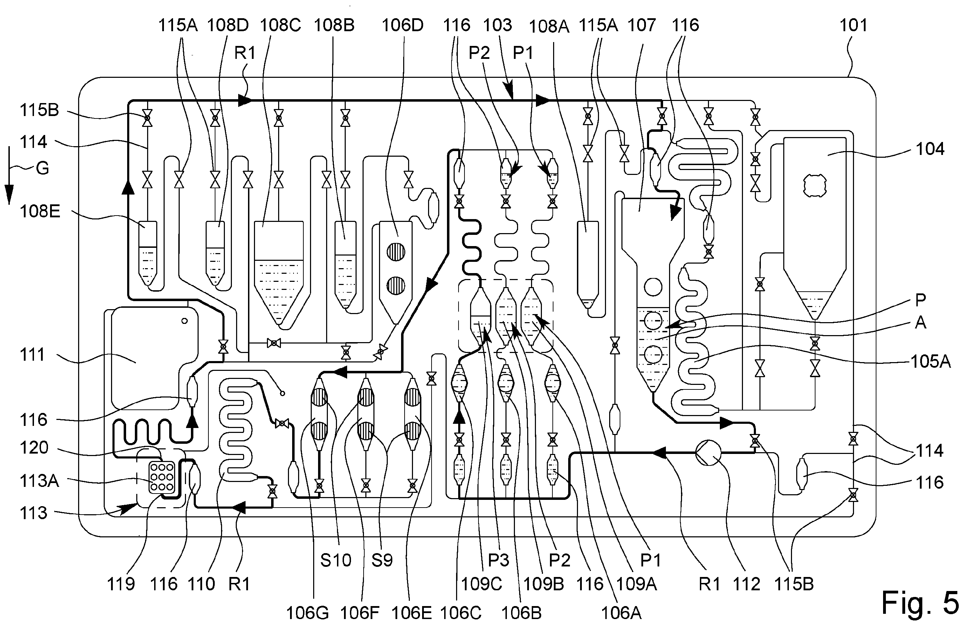

In the following, with reference to Fig. 5 to Fig. 8, the proposed method or individual method steps are explained in greater detail, some of the reference signs that are shown in Fig. 2 being omitted in these figures for reasons of clarity.

Preferably, in some or all of the method steps, different fluidic circuits, channels and/or cavities are generated or used in the fluid system 103 by activating the actuators 205 or valves 115 and/or the fluid flows through these different fluidic circuits, channels and/or cavities.

The fluidic circuit or channel in the fluid system 103 that is being used or is active in the respective method steps is highlighted in Fig. 5 to Fig. 8.

The method sequence, in particular the flow and conveying of the fluids, the mixing and the like, is controlled by the analysis device 200 or the control apparatus 207, in particular by accordingly activating and actuating the pump drive 202 or the pump apparatus 112 and/or the actuators 205 or valves 115.

Preferably, the pump apparatus 112 is integrated in the respective fluidic circuits used and/or generated, in particular by accordingly actuating the valves 114, and/or fluid flows through the pump apparatus 112 when said fluids are conveyed in the fluid system 103.

In particular, starting from the pump apparatus 112, the conveyed fluid flows in a circuit back to the pump apparatus 112 again. Particularly preferably, starting from the pump apparatus 112, a fluid, in particular the sample P or sample portions, is pumped into the respective channels and/or the respective cavities, with the fluid located therein being displaced.

Preferably, in the different method steps, the fluid, in particular the sample P, is not fully circulated in the respective circuits, but rather is only circulated until the cavity that is to be filled, or is necessary for the respective method steps, is (completely) filled, with the sensor portion 116, which is arranged (directly) downstream of or after the cavity, preferably detecting when the cavity has been (completely) filled.

In particular, the pump apparatus 112 is deactivated or no longer actuated, another circuit is activated or released by selectively opening and closing the valves 115, and/or the next method step is initiated, when the sensor portion 116 or sensor 206A arranged (directly) downstream of or after the cavity to be filled detects a flow of fluid or a liquid front. However, other solutions are also possible here, in particular those in which, additionally or alternatively, the start and/or end of the conveying and/or of the respective method steps is specified or fixed in time and/or on the basis of the number of steps and/or the rotational speed of the pump drive 202, in order to convey the fluid in the cartridge 100 in the desired manner.

Preferably, the conveying direction of the fluid varies in several method steps and/or the conveying direction is changed or reversed between several method steps.

The preferred conveying direction in the respective method steps is indicated by arrows in Fig. 5 to Fig. 8.

Preferably, the receiving cavity 104, the mixing cavity 107 and the pump apparatus 112 are initially interconnected to form a (first) fluidic circuit, in particular in order to pump the sample P from the receiving cavity 104 into the mixing cavity 107, in particular by means of the pump apparatus 112.

Preferably, the sample P or a part or supernatant of the sample P is removed from the receiving cavity 104 at the bottom or via the outlet 104C, preferably for carrying out the nucleic-acid assay, and/or centrally or via the intermediate connection 104D, in particular for carrying out the protein assay, and is preferably fed to the mixing cavity 107 in a metered manner.

Preferably, the sample P in the cartridge 100 is metered, in particular in or by means of the first metering cavity 105A and/or second metering cavity 105B, before being introduced into the mixing cavity 107. Here, in particular the upstream and/or downstream sensor portions 116 are used together with the assigned sensors 206 in order to make possible the desired metering. However, other solutions are also possible.

In the mixing cavity 107, the sample P is prepared for further analysis and/or is mixed with a reagent, preferably with a liquid reagent F1 from a first storage cavity 108A and/or with one or more dry reagents S1, S2 and/or S3, which are preferably provided in the mixing cavity 107.

The liquid and/or dry reagents can be introduced into the mixing cavity 107 before and/or after the sample P. Particularly preferably, the dry reagents S1 to S3 are introduced into the mixing cavity 107 previously or before the sample P and/or other fluids, such as the liquid reagent F1, are added, and said dry reagents are optionally dissolved by the sample P and/or other fluids, in particular the liquid reagent F1.

The liquid reagent F1 may be a reagent, in particular a PCR master mix for the amplification reaction or PCR, and/or may be a sample buffer. Preferably, the PCR master mix contains nuclease-free water, enzymes for carrying out the PCR, in particular at least one DNA polymerase, nucleoside triphosphates (NTPs), in particular deoxynucleotides (dNTPs), salts, in particular magnesium chloride, and/or reaction buffers.

The dry reagents S1, S2 and/or S3 may likewise be reagents required for carrying out an amplification reaction or PCR, which are in a dry, in particular lyophilised, form. Preferably, the dry reagents S1, S2 and/or S3 are selected in particular from lyophilised enzymes, preferably reverse transcriptases, DNA polymerases, NTPs, dNTPs and/or salts, preferably magnesium chloride.

The dissolving or mixing in the mixing cavity 107 takes place or is assisted in particular by introducing and/or blowing in gas or air, in particular from the bottom and/or via the outlet. This is carried out in particular by accordingly pumping gas or air in the circuit by means of the pump or pump apparatus 112.

Particularly preferably, the mixing cavity 107 and the pump apparatus 112 are interconnected in a (second) fluidic circuit in order to mix the sample P with one or more reagents. Preferably, gas or air is then removed from the top of the mixing cavity 107 and is fed to the mixing cavity 107 from the bottom by means of the pump apparatus 112, in particular such that the gas or air rises from the bottom to the top in the mixing cavity 107, and/or turbulence is generated in the mixing cavity 107.

As already described at the outset, the mixing cavity 107 preferably enlarges towards the top, in particular such that bubbles that form or collect in the mixing cavity 107 on the surface due to the mixing process remain in the mixing cavity 107 and do not penetrate adjacent cavities and/or channels. In particular, the cross-sectional area of the mixing cavity 107 that enlarges towards the top encourages the bubbles to burst, and therefore foam formation is reduced. In this way, there is enough time available for the mixing process.

Subsequently, in particular during the nucleic-acid assay, a desired volume of the sample P that is mixed and/or pretreated in the mixing cavity 107 is preferably fed to one or more reaction cavities 109, particularly preferably via (respectively) one of the optional intermediate cavities 106A to 106C arranged before or upstream of the respective reaction cavities 109 and/or with different reagents or primers, in this case dry reagents S4 to S6, being added or dissolved.

Particularly preferably, in particular during the nucleic-acid assay, the (premixed) sample P is split into several sample portions, preferably of equal size, and/or is divided between the intermediate cavities 106A to 106C and/or reaction cavities 109, preferably evenly and/or in sample portions of equal size.

Different reagents, in the present case dry reagents S4 to S6, particularly preferably primers, in particular those required for the PCR or PCRs, in particular groups of different primers in this case, are preferably added to the (premixed) sample P or the sample portions in the intermediate cavities 106A to 106C and/or different reaction cavities 109, respectively.

The primers in the different groups or sample portions differ in particular in terms of the hybridisation temperatures of the amplification products generated by the respective primers.

Particularly preferably, marker primers are used in the sense already specified at the outset.

In the embodiment shown, the reagents or primers S4 to S6 are contained in the intermediate cavities 106A to 106C. However, other solutions are also possible, in particular those in which the reagents or primers S4 to S6 are contained in the reaction cavities 109.

According to a preferred embodiment, the intermediate cavities 106A to 106C each contain primers for amplifying/copying one analyte A, preferably two different analytes A and more preferably three different analytes A. However, it is also possible for four or more different analytes A to be amplified/copied per reaction cavity 109 or sample portion.

Fig. 5 is a schematic view of the cartridge 100 when the reaction cavities 109 are being filled with the sample P and/or when the sample P is being divided into several sample portions, three in this case.

In the particularly preferred method variant shown, the sample P is divided into a first sample portion P1, a second sample portion P2 and an optional third sample portion P3, preferably by accordingly activating and actuating the pump drive 202 or pump apparatus 112 and/or the actuators 205 or valves 115.

Preferably, the sample portions are fed to different reaction cavities 109, in particular from below.

Preferably, the first reaction cavity 109A is filled with the first sample portion P1, the second reaction cavity 109B is filled with the second sample portion P2 and the optional third reaction cavity 109C is filled with the optional third sample portion P3.

Particularly preferably, the valves 115 that are assigned to the reaction cavities 109, and are in particular upstream and downstream, are sequentially opened, preferably such that the reaction cavities 109 can be individually or sequentially loaded with the sample P or the respective sample portions, and/or such that the sample P can be divided into a plurality of sample portions assigned to the reaction cavities 109.

Fig. 5 shows the state of the cartridge 100 and/or the method step in which the first reaction cavity 109A and the second reaction cavity 109B are already completely filled and the third reaction cavity 109C is being filled with the third sample portion P3.

Preferably, the reaction cavities 109 are filled by (continuously) pumping using the pump apparatus 112, in particular until the sample P or the corresponding sample portion reaches the sensor portion 116 arranged directly downstream or thereafter, and/or until a flow of fluid is detected in the sensor portion 116 arranged directly downstream or thereafter, as shown in Fig. 5 for the sensor portions 116 arranged downstream of the first reaction cavity 109A and the second reaction cavity 109B, respectively. This ensures that the reaction cavities 109 are completely filled, and/or that the next method step, in particular the amplification of the analytes A, can only be initiated once the reaction cavities 109 have been completely filled.

Furthermore, by means of the sensor portions 116 and/or sensors 206A it is possible to adapt the conveying speed of the fluid and/or the operation of the pump drive 202 for particular method steps and/or temporarily, in a targeted and direct manner. For example, by means of the sensor portions 116 and/or sensors 206A, arranged upstream of and/or before the reaction cavities 109 and/or intermediate cavities 106A to 106C, it is possible to adapt and/or reduce the conveying speed of the fluid for receiving the primers S4 to S6 in the intermediate cavities 106A to 106C immediately after a flow of fluid is accordingly detected, preferably such that a desired redissolving volume flow rate is set and/or it is ensured that the primers S4 to S6 completely dissolve.

As the reaction cavities 109 are being filled with the sample P or the corresponding sample portions, the fluid located in the reaction cavities 109, in particular the air located in the reaction cavities 109, is displaced and/or fed to a downstream cavity, for example the receiving cavity 104 or the mixing cavity 107. In the method variant shown, the (pretreated) sample P is removed from the bottom of the mixing cavity 107 and at the same time the fluid, in particular the air, displaced by the sample P or sample portions is fed to the mixing cavity 107 at the top, in particular until the reaction cavities 109 are completely filled with the sample P or the respective sample portions.

Preferably, the sample portions are handled or conveyed individually, independently and/or separately from one another in the remainder of the method sequence, as explained in greater detail in the following. However, other variants of the method are also possible in which the sample P is only temporarily divided into sample portions, and/or in which the sample portions are brought back together and are handled or conveyed together in the further method sequence.

Particularly preferably, the reaction cavities 109 are filled in succession with a specified volume of the (pretreated) sample P or with respective sample portions via the intermediate cavities 106A to 106C that are each arranged upstream of the respective reaction cavities 109. For example, the first reaction cavity 109A is filled with a specified volume of the pretreated sample P before the second reaction cavity 109B and/or the second reaction cavity 109B is filled therewith before the third reaction cavity 109C.

In the reaction cavities 109, the amplification reactions or PCRs are carried out to copy/amplify the analytes A or target nucleic-acid sequences. This is carried out in particular by means of the assigned, preferably common, reaction temperature-control apparatus(es) 204A and/or preferably simultaneously for all the reaction cavities 109, i.e. in particular using the same cycles and/or temperature (curves/profiles).

Preferably, analytes A of the sample portions are amplified in parallel and/or simultaneously in the different reaction cavities 109. However, other variants of the method are also possible here, in particular those in which the analytes A of the sample portions are amplified sequentially or in succession. For example, the analytes A of the first sample portion P1 can be amplified before the analytes A of the second sample portion P2.

The PCR or PCRs are carried out on the basis of protocols or temperature profiles that are essentially known to a person skilled in the art. In particular, the mixture or sample volume located in the reaction cavities 109 is preferably cyclically heated and cooled.

Preferably, nucleic-acid products and/or target nucleic-acid sequences are produced from the analytes A as amplification products in the reaction cavity/cavities 109.

During the nucleic-acid assay, a label L is in particular produced directly and/or during the amplification reaction(s) (in each case) and/or is attached to the analytes A, amplification products and/or target nucleic-acid sequences. This is in particular achieved by using corresponding, preferably biotinylated, primers. However, the label L can also be produced and/or bonded to the analytes A, amplification products, target nucleic-acid sequences and/or target proteins separately or later, optionally also only in the sensor compartment 118 and/or after hybridisation. In particular, during the protein assay, a label L is only bonded to the analytes A or target proteins after hybridisation of the analytes A or target proteins to the capture molecules M.

The label L is used in particular for detecting bonded analytes A or amplification products. In particular, the label L can be detected or the label L can be identified in a detection process, as explained in greater detail in the following.

Particularly preferably, it is provided for a plurality of amplification reactions or PCRs to be carried out in parallel or independently from one another using different primers S4 to S6 and/or primer pairs, such that a large number of (different) analytes A or target nucleic-acid sequences can be copied or amplified in parallel and subsequently analysed.

After carrying out the amplification reaction(s), corresponding fluid volumes, sample portions and/or amplification products are conducted out of the reaction cavities 109 in succession to the (common or same) sensor arrangement, in particular to the (common or same) sensor apparatus 113 and/or to the (common or same) sensor compartment 118, in particular via a group-specific and/or separate intermediate cavity 106E, 106F or 106G, (respectively) and/or via the optional (common) intermediate temperature-control cavity 110.

Particularly preferably, the sample portions are each individually, in particular sequentially, conducted to the (same) sensor arrangement, in particular to the sensor apparatus 113 and/or to the (precisely one and/or common) sensor compartment 118, in particular by accordingly activating the pump drive 202 or pump apparatus 112 and/or the actuators 205 or valves 115.

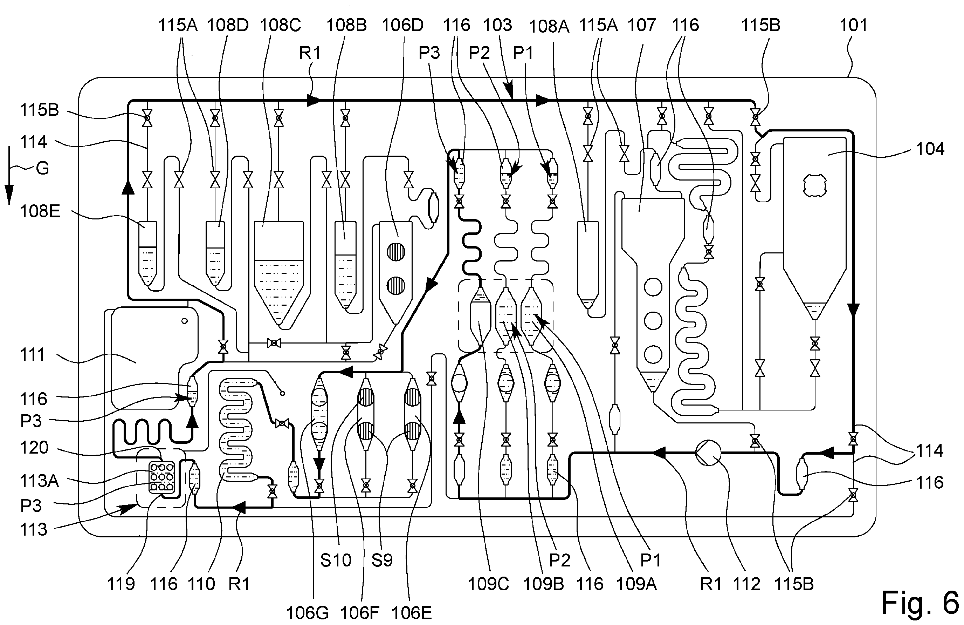

Fig. 6 is a schematic view of the cartridge 100 when one of the sample portions, in this case the third sample portion P3, is being conveyed to the sensor arrangement or sensor apparatus 113, in particular in order to bond analytes A of the sample portion, in this case the third sample portion P3, to the corresponding capture molecules M.

Preferably, after carrying out the amplification reaction(s), one of the sample portions, in this case initially the third sample portion P3, is conveyed to the sensor arrangement or sensor apparatus 113 or to the sensor compartment 118, in particular while the other sample portion(s), in this case the first sample portion P1 and the second sample portion P2, remain in the reaction cavities 109, as shown in Fig. 6.

In particular, once the amplification reaction(s) is/are complete, the reaction cavities 109 are sequentially and/or each individually emptied, fluid preferably flowing through the reaction cavities 109 from the bottom to the top for the purpose of emptying, and/or the sample portions preferably being pumped out of the reaction cavities 109 towards the top. Preferably, the reaction cavities 109 and/or the flow cross sections of the reaction cavities 109 are of such a (small) size that, in particular due to the capillary pressure or the adhesive force, the fluid is prevented from becoming detached from the walls and/or it is possible to pump out the fluid against gravity, as shown in Fig. 6 for the third reaction cavity 109C.

The reaction cavities 109 are preferably emptied by introducing a fluid, in particular air, into the reaction cavities 109, particularly preferably from the bottom. As shown, the sample P or sample portions is/are conveyed in a closed fluidic circuit, in particular sectionwise and/or from one cavity to the next or downstream cavity.

The sample portions are preferably fed to the sensor arrangement or sensor apparatus 113 or the sensor compartment 118 via different intermediate cavities. Particularly preferably, the first sample portion P1 is conducted via the first intermediate cavity 106E, the second sample portion P2 via the second intermediate cavity 106F and the optional third sample portion P3 via the optional third intermediate cavity 106G, in particular in order for each of said portions to be individually pretreated for the sensor arrangement or sensor apparatus 113.

The intermediate cavities 106E to 106G may contain further reagents, in this case dry reagents S9 and S10, respectively, for preparing the amplification products for the hybridisation, e.g. a buffer, in particular an SSC buffer, and/or salts for further conditioning. On this basis, further conditioning of the analytes A or amplification products can be carried out, in particular in order to improve the efficiency of the subsequent hybridisation (bonding to the capture molecules M). Particularly preferably, the pH of the sample P is set or optimised in the intermediate cavities 106E to 106G and/or by means of the dry reagents S9 and S10.

Optionally, the sample P or sample portions or the analytes A or amplification products is/are, in particular immediately before being fed to the sensor arrangement or sensor apparatus 113 and/or between the reaction cavities 109 and the sensor arrangement or sensor apparatus 113, actively temperature-controlled (in advance), preferably preheated, in particular by means of and/or in the intermediate temperature-control cavity 110 and/or by means of the intermediate temperature-control apparatus 204B, particularly preferably in order to denature the analytes A or amplification products.

When carrying out the protein assay, the sample P or the analytes A or the target proteins is/are preferably fed directly from the mixing cavity 107 to the sensor arrangement or sensor apparatus 113 and/or is/are guided past the intermediate cavity/cavities 106, reaction cavity/cavities 109 and/or the intermediate temperature-control cavity 110 via the bypass 114A.

The sample P or sample portions is/are fed to the sensor arrangement, sensor apparatus 113 and/or the sensor compartment 118, preferably in a first conveying direction R1, as indicated in Fig. 6 by arrows. In particular, the pump apparatus 112 is operated such that the sample P or sample portions is/are pumped in a first conveying direction R1 to the sensor arrangement, sensor apparatus 113 and/or the sensor compartment 118 and/or penetrate(s) the sensor compartment 118 via the inlet 119 and/or from the bottom.

Particularly preferably, when the sensor arrangement, in particular the sensor compartment 118, is being filled with the sample P or sample portions, fluid flows therethrough in the first conveying direction R1 and/or from the inlet 119 to the outlet 120 and/or vertically and/or from the bottom to the top.

Preferably, the sample portions are fed sequentially and/or each individually to the sensor arrangement or sensor apparatus 113 in particular via the inlet 119 and/or from the bottom, in particular in order to bond the analytes A of the respective sample portions to the corresponding capture molecules M of the sensor apparatus 113.

In particular, the analytes A of the sample portions are sequentially and/or individually bonded to the corresponding capture molecules M of the sensor apparatus 113, and the bonded analytes A of all the sample portions are identified, detected or determined together and/or in a single or common detection process, as explained in greater detail in the following.

Once the sensor arrangement, in particular the sensor compartment 118, has been (completely) filled with the sample P or one of the sample portions, the conveying is stopped and/or the analytes A are hybridised to the corresponding capture molecules M of the sensor apparatus 113, preferably by (actively) temperature-controlling, in particular heating, the sensor arrangement or sensor apparatus 113, in particular by means of the sensor temperature-control apparatus 204C.

For the hybridisation of the analytes A, the sample P or sample portions is/are each kept in the sensor arrangement or on the sensor apparatus 113 or in the sensor compartment 118 for a certain length of time. In particular, the pump stops conveying or operating for a certain length of time, in particular such that the sample P or sample portions is/are each retained in the sensor arrangement or on the sensor apparatus 113 for the hybridisation.

Preferably, the sample P or sample portions is/are each kept in the sensor arrangement or on the sensor apparatus 113 or in the sensor compartment 118 for more than 10 seconds or 30 seconds, particularly preferably more than 60 seconds or 120 seconds, and/or for less than 10 minutes or 8 minutes, particularly preferably less than 5 minutes. This ensures that, in particular enough, analytes A are bonded to corresponding capture molecules M.

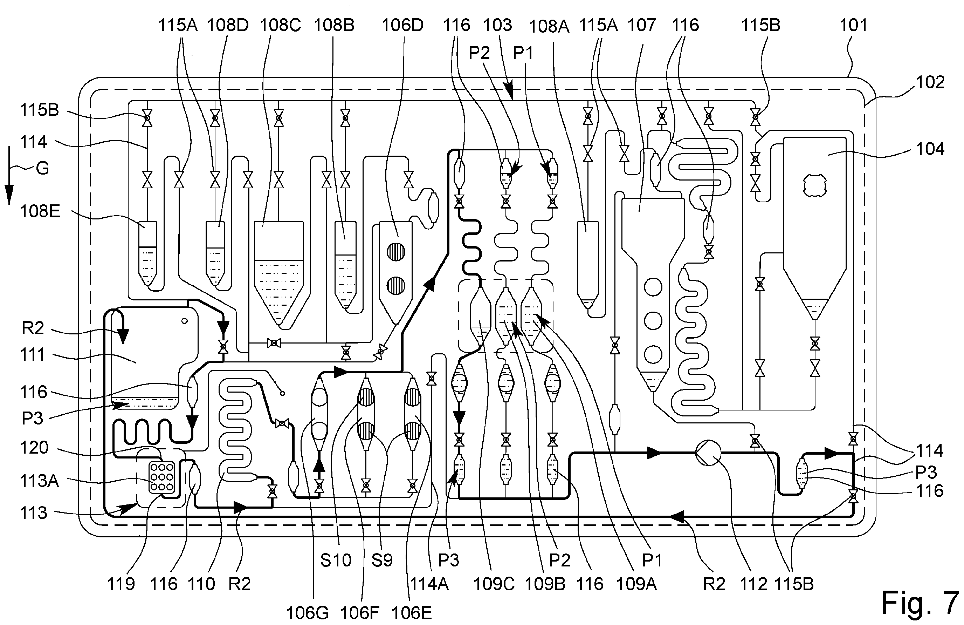

Fig. 7 is a schematic view of the cartridge 100 when the sensor arrangement or sensor compartment 118 is subsequently being emptied, and/or when one of the sample portions, in this case the third sample portion P3, is being conveyed away.

Preferably, the sample portions are carried away from the sensor arrangement or sensor apparatus 113 sequentially, in particular after the analytes A have bonded to the corresponding capture molecules M, and/or are pumped out of the sensor arrangement or the sensor compartment 118 and/or are fed to the (common) collection cavity 111.

In particular, the sample portions are fed sequentially or each individually to the sensor arrangement or sensor apparatus 113, their analytes A are bonded or hybridised there, as required, and then the portions are sequentially and/or each individually carried away from the sensor arrangement or sensor apparatus 113, in particular before another of the sample portions is fed to the sensor arrangement or sensor apparatus 113 for the hybridisation. For example, the third sample portion P3 is fed to the sensor arrangement or sensor apparatus 113 and then is carried away from the sensor arrangement or sensor apparatus 113 before the second sample portion P2 is fed to the sensor arrangement or sensor apparatus 113 and then is carried away from the sensor arrangement or sensor apparatus 113.

Preferably, the second sample portion P2 is fed to the sensor arrangement or sensor apparatus 113 and then is carried away from the sensor arrangement or sensor apparatus 113 before the first sample portion P1 is fed to the sensor arrangement or sensor apparatus 113 and then is carried away from the sensor arrangement or sensor apparatus 113.

Particularly preferably, the sensor arrangement, in particular the sensor compartment 118, is emptied once the analytes A have bonded to the corresponding capture molecules M, and/or the sample P or sample portion is displaced, in particular from the top, by means of a gas, such as air, in particular taken from the collection cavity 111. However, variants of the method are also possible in which the sample P or sample portions is/are displaced or carried away from the sensor arrangement or the sensor compartment 118 by means of another fluid, for example the wash buffer from the storage cavity 108C.

In particular, variants of the method are also possible in which one of the sample portions, for example the second sample portion P2, is displaced out of the sensor arrangement or the sensor compartment 118 by one of the other sample portions, for example the first sample portion P1, and/or in which the sample portion located in the sensor arrangement or on the sensor apparatus 113 is displaced out of the sensor arrangement or sensor apparatus 113 by the subsequent sample portion being fed in.

Preferably, the conveying direction is reversed after hybridisation. In particular, the sample P or sample portions is/are carried away from the sensor arrangement or sensor apparatus 113 in a second conveying direction R2 which is opposite to the first conveying direction R1, as indicated in Fig. 7 by arrows.

It is therefore preferable for the sensor arrangement or the sensor compartment 118 to be loaded or filled in one conveying direction with a fluid, in particular the sample P or sample portions, and then to be emptied in a different, in particular opposite, conveying direction. Preferably, the sensor arrangement or the sensor compartment 118 is loaded or filled with the sample P or sample portions in the first conveying direction R1 and then subsequently is emptied and/or is loaded or filled with a gas, in particular air, in the second conveying direction R2, in particular in order to displace the sample P or sample portion out of the sensor arrangement or the sensor compartment 118.

Particularly preferably, during emptying, fluid flows through the sensor arrangement, in particular the sensor compartment 118, in the second conveying direction R2 and/or from the outlet 120 to the inlet 119 and/or vertically and/or from the top to the bottom, said fluid in particular being a gas or air from the collection cavity 111.

Preferably, in particular in addition to the sensor arrangement or the sensor compartment 118, the channels or channel portions and the cavities between the sensor arrangement or sensor apparatus 113 and the reaction cavities 109 are flushed and/or emptied after hybridisation. Advantageously, the next of the sample portions, in this case the second sample portion P2, can then be fed to the sensor arrangement or sensor apparatus 113 via the channels and/or cavities that have in particular been emptied in this way. Particularly preferably, residues of the used sample portion do not remain between the sensor arrangement or sensor apparatus 113 and the reaction cavities 109.

As already explained, in particular the collection cavity 111 is used to empty the sensor arrangement or the sensor compartment 118. Preferably, the collection cavity 111 receives the used sample portion, in this case the third sample portion P3, and simultaneously provides a gas, preferably air, for emptying the sensor arrangement and/or the sensor compartment 118.

Preferably, for this purpose the collection cavity 111, the pump apparatus 112 and the sensor arrangement or the sensor compartment 118 are interconnected in a fluidic circuit, in particular by accordingly actuating the valves 115.

Particularly preferably, a gas, in particular air, or another fluid is discharged from the collection cavity 111 towards the top in the normal operating position of the cartridge 100, in particular such that the sample P or sample portion received or collected in the collection cavity 111 cannot penetrate the fluidic circuit or be fed into the sensor arrangement or sensor apparatus 113 again. In particular, the used sample P or sample portion is (finally) disposed of by involving or using the collection cavity 111.

After hybridising and/or bonding the sample P, analytes A and/or amplification products to the capture molecules M, and/or after collecting all the sample portions in the collection cavity 111, the sensor arrangement and/or sensor apparatus 113 and/or the bonded analytes A are pretreated for the detection, in particular by means of fluids from the storage cavities 108B to 108E.

Preferably, the sensor arrangement or sensor apparatus 113 is prepared or pretreated for the detection of the bonded analytes A after hybridising the analytes A of all the sample portions and/or after collecting all the sample portions in the collection cavity 111.

Preferably, the pretreatment of the sensor arrangement or sensor apparatus 113 that follows the hybridisation only takes place once all the sample portions have been fed to the sensor arrangement or sensor apparatus 113 and have then been carried away from the sensor arrangement or sensor apparatus 113 and/or collected or disposed of in the collection cavity 111.

Preferably, in particular after the analytes A have bonded to the corresponding capture molecules M and/or before the bonded analytes A have been detected, for the detection the sensor arrangement or sensor apparatus 113 is pretreated or flushed with one or more fluids, in particular a wash buffer and/or a reagent, particularly preferably from the storage cavities 108.

Preferably, for the pretreatment, a fluid, in particular a reagent and/or wash buffer, is fed to the sensor arrangement or sensor apparatus 113 via the outlet 120 and/or from the top and/or in the second conveying direction R2, in order to flush the sensor arrangement and/or sensor compartment 118.

In particular, the sample P or sample portions and a fluid, in particular a reagent and/or wash buffer, are fed to the sensor arrangement or sensor apparatus 113 from different sides, the sample P or sample portions preferably being fed to the sensor arrangement or sensor apparatus 113 via the inlet 119 and/or from the bottom and/or in the first conveying direction R1, and the fluid, in particular a reagent and/or the wash buffer, preferably being fed to said sensor arrangement or sensor apparatus 113 for the pretreatment via the outlet 120 and/or from the top and/or in the second conveying direction R2.

Preferably, after bonding the analytes A and/or removing the (last) sample portion from the sensor arrangement, an optional washing process is carried out and/or other reagents or liquids are optionally, preferably sequentially, fed in, in particular from the storage cavities 108B to 108E.

As already explained, in the initial state of the cartridge 100 or when at the factory, the storage cavities 108 are preferably filled at least in part, in particular with a fluid such as a reagent, solvent or wash buffer, in particular for the pretreatment and subsequent detection.

Preferably, for the pretreatment, the collection cavity 111, the pump apparatus 112, the sensor arrangement, sensor apparatus 113 and one of the storage cavities 108, respectively, are interconnected in a fluidic circuit, in particular by accordingly actuating the valves 115, in particular in order to feed the fluid from the respective storage cavities 108 to the sensor arrangement or sensor apparatus 113 and/or via the sensor arrangement or sensor apparatus 113 to the collection cavity 111.

It is preferable for the fluids contained in the storage cavities 108, at least in the normal operating position of the cartridge 100, to be removed or pumped out at the bottom and/or at the outlet, with a fluid, in particular a gas, particularly preferably from the collection cavity 111, preferably flowing in at the top and/or at the inlet for pressure equalisation. In particular, fluid flows through the storage cavities 108 vertically, in particular from the top to the bottom, in order for said cavities to be emptied and/or for the fluid contained therein to be released. In this way, gas is not pumped out and foam formation is counteracted.

In particular, it may be provided that, in a washing process, remnants of the sample P or sample portions, in particular unbonded analytes A, amplification products, reagents or remnants from the PCR, and/or other substances that may disrupt the remainder of the method sequence, are in particular removed from the sensor compartment 118 and/or from the sensor apparatus 113, preferably by means of a fluid or reagent F3 from the storage cavity 108C.

Particularly preferably, a washing process for the sensor arrangement or sensor apparatus 113 is an optional process and/or method step in which a fluid, in particular a wash buffer, is conveyed through the sensor compartment 118 and/or is conducted past the sensor apparatus 113, in particular in order to wash away or flush out unbonded analytes A, sample residues or other remnants from the sensor compartment 118 and/or the region of the sensor apparatus 113.

Washing, flushing or the washing process may in particular take place using a fluid or reagent F3, in particular a wash buffer, particularly preferably a sodium-citrate buffer or SSC buffer, which is preferably contained in the storage cavity 108C. Unbonded analytes A and/or amplification products and substances which could disrupt or impair subsequent detection are preferably removed from the sensor compartment 118 and/or from the sensor apparatus 113 by the wash buffer and/or fed to the collection cavity 111.

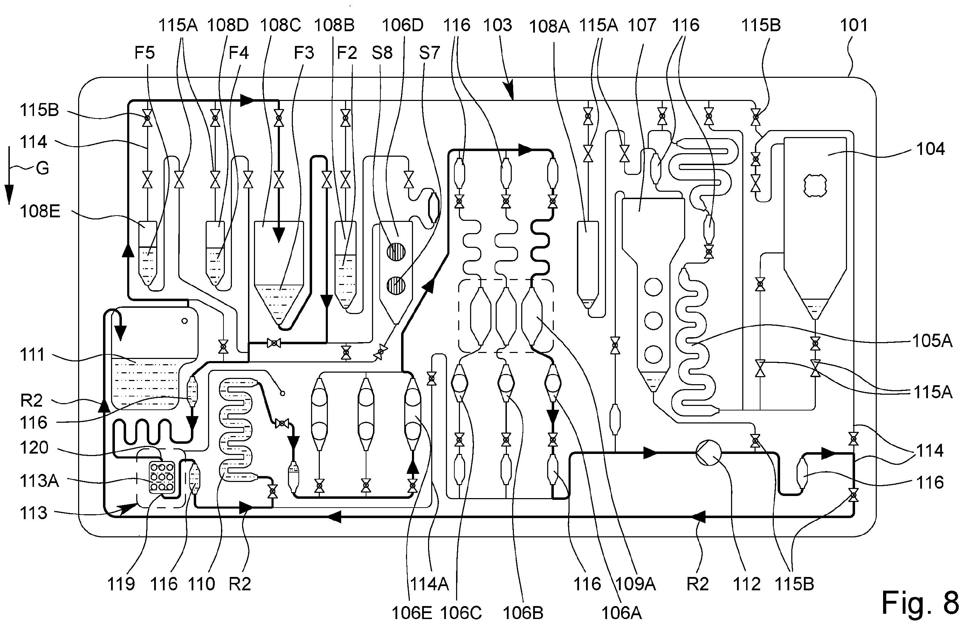

Fig. 8 is a schematic view of the cartridge 100 during the washing process and/or when the sensor arrangement or sensor apparatus 113 is being flushed by means of the wash buffer or reagent F3 from the storage cavity 108C.

Preferably, for pretreatment and/or when flushing the sensor arrangement or sensor apparatus 113, in particular using the wash buffer, the sensor cover 117 is actuated and/or moved relative to the sensor apparatus 113 and/or at least temporarily lowered onto the sensor apparatus 113. Preferably, for this purpose the conveying by means of the pump drive 202 is stopped. However, variants of the method are also possible in which the sensor cover 117 is lowered onto the sensor apparatus 113 when the fluid is flowing through and/or during conveying.

The sensor cover 117 is preferably pneumatically actuated and/or lowered by means of compressed air, the compressed air preferably being provided by the analysis device 200, in particular the pressurised gas supply 214, and/or being fed to the cartridge 100.

Particularly preferably, the sensor cover 117 is lowered onto the sensor apparatus 113 within a defined period of time and is pressed onto the sensor apparatus 113 and/or kept on the sensor apparatus 113 for a time period of more than 1 second or 2 seconds, in particular more than 3 seconds or 4 seconds, and/or less than 60 seconds or 30 seconds, in particular less than 20 seconds or 10 seconds. However, variants of the method are also possible in which the sensor cover 117 is actuated in a pulsed or abrupt or impulsive manner.

In particular, in the washing process, the sensor arrangement and/or the sensor compartment 118 is initially filled or loaded with the wash buffer, in particular from the top and/or via the outlet 120, and then the sensor cover 117 is lowered onto the sensor apparatus 113, in particular in order to flush the sensor apparatus 113 or the individual sensor fields 113B and/or to remove or dissipate air bubbles, remnants or the like. This increases the efficiency of the pretreatment, in particular of the washing process. Preferably, the wash buffer is then fed to the collection cavity 111 from the sensor arrangement or sensor apparatus 113, in particular in the second conveying direction R2.

Subsequently and/or after the washing process, in accordance with a preferred variant of the method, there are additional method steps for preparing the detection of the analytes A or amplification products bonded to the capture molecules M.

In the following, the particularly preferred variant of the detection is described in greater detail, specifically electrochemical detection or detection by means of redox cycling, but other types of detection, for example optical or capacitive detection, may also be carried out.

If the bonded analytes A or amplification products are still not marked or provided with a label L, in particular during the protein assay, labels L are then fed to the sensor arrangement or the sensor compartment 118, preferably from the storage cavity 108E, particularly preferably in the form of a liquid reagent F5. Optionally, there is then another washing process, the sensor cover 117 preferably being actuated or used (again).

In order to detect the analytes A or amplification products bonded to the capture molecules M, a reagent F4 and/or detector molecules D, in particular alkaline phosphatase/streptavidin, is/are fed to the sensor arrangement or sensor apparatus 113, preferably from the storage cavity 108D.

Particularly preferably, the reagent F4 and/or the detector molecules D is/are fed to the sensor arrangement via the outlet 120 and/or from the top and/or in the second conveying direction R2 for the detection or during pretreatment. In particular, the reagent F4 and/or the detector molecules D and the sample P or sample portions is/are fed to the sensor arrangement or sensor apparatus 113 from different sides.

Within the meaning of the present invention, the term "detector molecules" is preferably understood to mean molecules that bond specifically to the marker or label L of the (bonded) analytes A or amplification products and thus allow the detection thereof.

In particular, the detector molecules D may be enzyme conjugates and/or immunoconjugates, which bond specifically to the marker or label L, in particular biotin, and comprise a reporter enzyme for converting a substrate SU.

In the context of the present invention, the detector molecules D are preferably based on streptavidin, which has a high affinity for biotin, and/or alkaline phosphatase, which can convert non-reactive phosphate monoesters to electrochemically active molecules and phosphate.

Preferably, a detection system is used, where the label L is based on biotin and where the detector molecules D are based on streptavidin/alkaline phosphatase. However, other detector molecules D can also be used.

The reagents F4 or detector molecules D can bond to the bonded analytes A or amplification products, in particular to the label L of the bonded analytes A or amplification products, particularly preferably to the biotin marker, as shown in Fig. 3 and Fig. 4.

Preferably, the sensor cover 117 is actuated (again) and/or is at least temporarily lowered onto the sensor apparatus 113 when the sensor compartment 118 is filled with the reagent F4 or the detector molecules D. In this way, the sensor fields 113B are flushed with the reagent F4 and/or the detector molecules D, and/or the detector molecules D are divided between the sensor fields 113B such that the bonding of the detector molecules D and analytes A or labels L is optimised.

Preferably, the sensor cover 117 is lowered onto the sensor apparatus 113 for a certain length of time, in particular in order to provide enough time for bonding. Particularly preferably, the sensor cover 117 is pressed onto the sensor apparatus 113 for more than 10 seconds or 30 seconds, in particular more than 1 minute or 2 minutes, and/or for less than 10 minutes or 8 minutes, in particular less than 5 minutes, in order to bond the detector molecules D and the analytes A or labels L to one another.

Optionally, subsequently or after the reagents F4 and/or detector molecules D have bonded to the analytes A or amplification products or the labels L, an (additional) washing process and/or flushing takes place, preferably by means of the fluid or reagent F3 or wash buffer, in particular in order to remove unbonded reagents F4 and/or detector molecules D from the sensor arrangement and/or the sensor compartment 118. Preferably, in this case, the sensor cover 117 is used or actuated (again), in particular in order to remove or dissipate any bubbles, remnants or the like.

Therefore, in the preferred variant of the method, it is provided for the sensor cover 117 to be lowered multiple times, in particular during the washing process and when loading the sensor arrangement or the sensor compartment 118 with the reagent F4 or the detector molecules D, for the pretreatment or during the pretreatment and/or before the bonded analytes A are (actually) detected. In particular, a plurality of method steps for pretreatment are assisted by actuating and/or lowering the sensor cover 117.

Preferably, when actuating the sensor cover 117, at least one valve 115, which is preferably arranged upstream or downstream of the sensor arrangement, is opened, in particular in order to allow pressure equalisation and/or to compensate for the pressure increase in the fluid system 103 that arises due to the sensor cover 117 being actuated. Particularly preferably, the sensor arrangement and/or the sensor compartment 118 is fluidically connected to a cavity filled with a gas, in particular air, in particular the collection cavity 111, in order to allow pressure equalisation.

Preferably, the reagent F4 and/or the (unbonded) detector molecules D is/are conveyed to the collection cavity 111, in particular in the second conveying direction R2. In particular, some or all of the channels, channel portions, cavities and/or sensor portions 116 of the (active) fluidic circuit are emptied (again), preferably by means of a gas, in particular air, from the collection cavity 111, as already explained.

It is therefore preferable, after several or each or all of the method steps and/or between several or each or all of the method steps, to empty several or all of the sensor portions 116, in particular the sensor portions 116 arranged directly upstream or downstream of the sensor arrangement, and/or it is preferable for a gas, in particular air, preferably from the collection cavity 111, the intermediate cavity 106D and/or from channels or channel portions, to flow through several or all of said sensor portions 116, in particular such that the fluid sensors 206A assigned to the sensor portions 116 can detect a flow of fluid or a liquid front in the following method step.

Preferably, a reagent S7 and/or S8 and/or substrate SU for the detection, in particular from the storage cavity 106D, is then fed to the sensor arrangement or sensor apparatus 113, preferably together with a fluid or reagent F2 (in particular a buffer), which is suitable for the substrate SU, particularly preferably for dissolving the reagent S7 and/or S8 and/or substrate SU, the fluid or reagent F2 in particular taken from the storage cavity 108B. In particular, the reagent S7 and/or S8 can form or can comprise the substrate SU.

Preferably, p-aminophenyl phosphate (pAPP) is used as the substrate SU.

The substrate SU preferably reacts on and/or with the bonded analytes A or amplification products and/or detector molecules D and/or allows these to be electrochemically measured.

In order to carry out the (actual) detection or electrochemical measurement of the bonded analytes A or amplification products or after adding the substrate SU, the sensor cover 117 is preferably pneumatically actuated or lowered onto the sensor apparatus 113, in particular in order to fluidically separate the (individual) sensor fields 113B from one another, and/or to prevent or minimise the exchange of substances between the sensor fields 113B.

By actuating or lowering the sensor cover 117, the diffusion paths of the (electrochemically active) molecules required for the measurement are reduced, in particular such that the measurement signal generated by the individual sensor fields 113B, which are fluidically separated from one another, is increased. In particular, a reaction and/or detection is prevented from being assigned to an incorrect or adjacent sensor field 113B, and in this way measurement inaccuracies or errors are prevented from occurring. In particular, the sensor cover 117 increases the measurement accuracy of the method.

Preferably, the sensor cover 117 is pressed onto the sensor apparatus 113 for more than 1 second or 2 seconds, in particular more than 5 seconds or 7 seconds, and/or for less than 10 minutes or 5 minutes, in particular less than 4 minutes or 2 minutes, in particular in order to provide enough time for the detection.

As shown in Fig. 4, the substrate SU is preferably split by the bonded detector molecules D, in particular the alkaline phosphatase of the bonded detector molecules D, preferably into a first substance SA, such as p-aminophenol, which is in particular electrochemically active and/or redox active, and a second substance SP, such as phosphate.

Preferably, the first or electrochemically active substance SA is detected in the sensor apparatus 113 or in the individual sensor fields 113B by electrochemical measurement and/or redox cycling.

Particularly preferably, by means of the first substance SA, a redox reaction takes place at the electrodes 113C, the first substance SA preferably discharging electrons to or receiving electrons from the electrodes 113C.

In particular, the presence of the first substance SA and/or the respective amounts in the respective sensor fields 113B is detected by the associated redox reactions. In this way, it can be determined qualitatively and in particular also quantitatively whether and how many analytes A or amplification products are bonded to the capture molecules M in the respective sensor fields 113B. This accordingly gives information on which analytes A are or were present in the sample P or sample portions, and in particular also gives information on the quantity of said analytes.

In particular, by means of the redox reaction with the first substance SA, an electrical power signal is generated at the assigned electrodes 113C, the power signal preferably being detected by means of an assigned electronic circuit.

Depending on the power signal from the electrodes 113C that is generated in this way, it is determined whether and/or where hybridisation to the capture molecules M has occurred.

The measurement is preferably taken just once and/or for the entire sensor array 113A and/or for all the sensor fields 113B, in particular simultaneously or in parallel. In particular, the bonded analytes A or amplification products are detected, identified or determined simultaneously or in parallel in a single or common detection process.

In particular, the bonded analytes A of all the sample portions are measured, identified, detected and/or determined together and/or in a single or common detection process.

However, in principle, it is also possible to measure a plurality of sample portions in the sensor apparatus 113 or in a plurality of sensor apparatuses 113 in succession and/or sequentially and/or separately.

The test results or measurement results, in particular of the protein assay or nucleic-acid assay, are in particular electrically transmitted to the analysis device 200 or the control apparatus 207 thereof, preferably by means of the electrical connection apparatus 203 and/or sequentially or simultaneously, and are accordingly prepared, analysed, stored, displayed and/or output, in particular by the display apparatus 209 and/or interface 210.

After the test has been carried out, the cartridge 100 is disconnected from the analysis device 200 and/or is released and/or ejected therefrom, and is in particular disposed of.

Individual aspects and features of the present invention and individual method steps and/or method variants may be implemented independently from one another, but also in any desired combination and/or order.

In particular, the present invention relates to any one of the following aspects which can be realized independently or in any combination, also in combination with any aspects above:
1. Method for testing an in particular biological sample (P),
   the sample (P) being received in a cartridge (100),
   the sample (P) being conveyed through a fluid system (103) with a plurality of channels (114) of the cartridge (100),
   the sample (P) being conveyed to a sensor arrangement of the cartridge (100) in order to detect analytes (A) of the sample (P),
   characterised
   in that the sensor arrangement is pretreated for detecting the analytes (A), a sensor cover (117) of the sensor arrangement being at least temporarily lowered onto a sensor apparatus (113) of the sensor arrangement both for pretreatment and for detection, and/or
   in that the sample (P) is divided into a plurality of sample portions (P1, P2, P3), the sample portions (P1, P2, P3) each being individually conveyed to the sensor arrangement, and/or
   in that the sample (P) or sample portions (P1, P2, P3) is/are conveyed to the sensor arrangement in a first conveying direction (R1) and then carried away from the sensor arrangement in a second conveying direction (R2) which is opposite to the first conveying direction (R1).
2. Method according to aspect 1, characterised in that the sample (P) is divided between different reaction cavities (109) and/or the sample portions (P1, P2, P3) are fed to different reaction cavities (109), analytes (A) of the sample (P) or sample portions (P1, P2, P3) preferably being amplified by means of amplification reactions, in particular PCR, in the different reaction cavities (109), preferably in parallel and/or independently from one another.
3. Method according to aspect 2, characterised in that the analytes (A) or sample portions (P1, P2, P3) are actively temperature-controlled between the reaction cavities (109) and the sensor arrangement, preferably in an intermediate temperature-control cavity (110).
4. Method according to any of the preceding aspects, characterised in that the analytes (A) of the sample (P) or sample portions (P1, P2, P3) are bonded to capture molecules (M) of the sensor arrangement and/or sensor apparatus (113) and that the bonded analytes (A) are detected by means of the sensor arrangement and/or sensor apparatus (113), preferably electrochemically and/or by redox cycling.
5. Method according to any of the preceding aspects, characterised in that the sample portions (P1, P2, P3) are fed to the sensor arrangement sequentially and/or in the first conveying direction (R1), in particular in order to bond the analytes (A) of the sample portions (P1, P2, P3) to the corresponding capture molecules (M).
6. Method according to any of the preceding aspects, characterised in that, in particular after the analytes (A) have bonded to the corresponding capture molecules (M), the sample portions (P1, P2, P3) are carried away from the sensor arrangement sequentially and/or in the second conveying direction (R2) which is opposite to the first conveying direction (R1), in particular in order to collect the sample portions (P1, P2, P3) in a collection cavity (111).
7. Method according to any of the preceding aspects, characterised in that the sample (P) or sample portions (P1, P2, P3) and a pretreatment fluid, in particular a reagent and/or wash buffer, are fed to the sensor arrangement from different sides, and/or in that the sample (P) or sample portions (P1, P2, P3) and/or a fluid for pretreatment, in particular a reagent and/or wash buffer, is/are conveyed from the sensor arrangement to a common collection cavity (111) of the cartridge (100), in particular in the second conveying direction (R2).
8. Method according to any of the preceding aspects, characterised in that, in particular after the analytes (A) have bonded to the corresponding capture molecules (M) and/or before the bonded analytes (A) have been detected, the sensor arrangement is pretreated and/or flushed with a fluid, in particular a wash buffer and/or a reagent, for the detection.
9. Method according to any of the preceding aspects, characterised in that the sensor arrangement is flushed with a wash buffer and/or is loaded with detector molecules (D) and/or a substrate (SU) for detecting the bonded analytes (A), and/or in that the sensor arrangement is flushed with the wash buffer multiple times, in particular after and/or during a plurality of method steps.
10. Method according to any of the preceding aspects, characterised in that the sensor cover (117) is pneumatically actuated and/or is lowered onto the sensor apparatus (113) multiple times, in particular after and/or during a plurality of method steps.
11. Method according to any of the preceding aspects, characterised in that the sensor cover (117) is actuated and/or lowered onto the sensor apparatus (113), in particular multiple times, for the pretreatment and/or before detection, in particular in order to flush sensor fields (113B) of the sensor apparatus (113) and/or to remove or dissipate air bubbles from the sensor apparatus (113), and/or in that the sensor cover (117) is lowered onto the sensor apparatus (113) for the detection, in particular in order to seal and/or fluidically separate sensor fields (113B) of the sensor apparatus (113) from one another and/or to reduce the diffusion paths of electrochemically active molecules in sensor fields (113B).
12. Method according to any of the preceding aspects, characterised in that the bonded analytes (A) of the sample (P) or sample portions (P1, P2, P3) are detected or determined in a single or common detection process, preferably when the sensor cover (117) is lowered.
13. Method according to any of the preceding aspects, characterised in that the cartridge (100) containing the sample (P) is received at least in part by an analysis device (200), the analysis device (200) preferably being pneumatically, thermally and/or electrically connected to the cartridge (100), and/or in that nucleic-acid sequences or proteins are detected as analytes (A) of the sample (P) or sample portions (P1, P2, P3).
14. Cartridge (100) for testing an in particular biological sample (P), the cartridge (100) comprising a fluid system (103) having a plurality of channels (114) and cavities, a pump apparatus (112) for conveying the sample (P) and/or a fluid, and a plurality of valves (114) for controlling the flow of the sample (P) and/or of the fluid through the fluid system (103),
   characterised
   in that different fluidic circuits can be formed in the fluid system (103) by actuating the valves (114), the pump apparatus (112) being integrated in all the circuits for conveying the sample (P) and/or the fluid, and/or
   in that one of the cavities is designed as a collection cavity (111), both the collection cavity (111) and pump apparatus (112) and at least one other of the cavities being interconnected or interconnectable in a fluidic circuit in order to convey a fluid out of the other of the cavities, and/or
   in that the cartridge (100) comprises a receiving cavity (104) for receiving the sample (P) and a mixing cavity (107) for mixing the sample (P) with a reagent, the receiving cavity (104), the mixing cavity (107) and the pump apparatus (112) being interconnected or interconnectable in a first fluidic circuit such that the sample (P) can be conveyed from the receiving cavity (104) into the mixing cavity (107) by means of the pump apparatus (112), and the mixing cavity (107) and the pump apparatus (112) being interconnected or interconnectable in a second fluidic circuit such that a gas can be drawn out of the mixing cavity (107) at the top by means of the pump apparatus (112) and can be conveyed into the mixing cavity (107) at the bottom by means of the pump apparatus (112), in order to mix the sample (P) with a reagent, and/or
   in that the cartridge (100) is designed to carry out the method according to any of the preceding aspects.
15. Cartridge according to aspect 14, characterised
   in that the cartridge (100) comprises a sensor arrangement for in particular electrochemically detecting analytes (A) of the sample (P), and/or
   in that a plurality of the cavities are designed as storage cavities (108), the storage cavities (108) each containing a fluid, in particular a reagent and/or a wash buffer, the collection cavity (111), the pump apparatus (112) and the sensor arrangement together with one of the storage cavities (108) being interconnected or interconnectable in a fluidic circuit in order to feed the fluid to the sensor arrangement from the respective storage cavities (108), and/or
   in that the collection cavity (111), the pump apparatus (112) and the sensor arrangement are interconnected or interconnectable in a fluidic circuit in order to feed a fluid, in particular a gas, to the sensor arrangement from the collection cavity (111) and/or to feed a fluid, in particular a sample residue and/or used reagents to the collection cavity (111) from the sensor arrangement, and/or
   in that in the delivery state of the cartridge (100) at least one reagent is in the mixing cavity (107) in order to pretreat the sample (P), and/or
   in that the cartridge (100) and/or the fluid system (103), in particular each of the fluidic circuits, are designed as a fluidically closed system.

### List of reference signs:

- 1: analysis system

- 100: cartridge
- 101: main body
- 102: cover
- 103: fluid system
- 104: receiving cavity
- 104A: connection
- 104B: inlet
- 104C: outlet
- 104D: intermediate connection
- 105: metering cavity
- 105A: first metering cavity
- 105B: second metering cavity
- 106(A-G): intermediate cavity
- 107: mixing cavity
- 108(A-E): storage cavity
- 109: reaction cavity
- 109A: first reaction cavity
- 109B: second reaction cavity
- 109C: third reaction cavity
- 110: intermediate temperature-control cavity
- 111: collection cavity
- 112: pump apparatus
- 113: sensor apparatus
- 113A: sensor array
- 113B: sensor field
- 113C: electrode
- 113D: support
- 113E: contact
- 113F: layer
- 114: channel
- 114A: bypass
- 115: valve
- 115A: initially closed valve
- 115B: initially open valve
- 116: sensor portion
- 117: sensor cover
- 118: sensor compartment
- 119: inlet
- 120: outlet

- 200: analysis device
- 201: receptacle
- 202: pump drive
- 203: connection apparatus
- 203A: contact element
- 204: temperature-control apparatus
- 204A: reaction temperature-control apparatus
- 204B: intermediate temperature-control apparatus
- 204C: sensor temperature-control apparatus
- 205: (valve) actuator
- 205A: (valve) actuator for 115A
- 205B: (valve) actuator for 115B
- 206: sensor
- 206A: fluid sensor
- 206B: other sensor
- 207: control apparatus
- 208: input apparatus
- 209: display apparatus
- 210: interface
- 211: power supply
- 211A: connection
- 212: housing
- 213: opening
- 214: pressurised gas supply
- 214A: connection element
- 215: support apparatus
- 215A: support element

- A(1-2): analyte
- B: bond
- D: detector molecule
- F(1-5): liquid reagent
- G: gravity
- H: main plane of extension
- L: label
- M(1-3): capture molecule
- P: sample
- P1: first sample portion
- P2: second sample portion
- P3: third sample portion
- R1: first conveying direction
- R2: second conveying direction
- S(1-10): dry reagent
- SU: substrate
- SA: first substance
- SP: second substance

## Claims

1. Method for testing an in particular biological sample (P),
the sample (P) being received in a cartridge (100),
the sample (P) being conveyed through a fluid system (103) with a plurality of channels (114) of the cartridge (100),
the sample (P) being conveyed to a sensor arrangement of the cartridge (100) in order to detect analytes (A) of the sample (P),
**characterised**
**in that** the sample (P) is divided into a plurality of sample portions (P1, P2, P3), wherein the sample portions (P1, P2, P3) are fed sequentially to the sensor arrangement in order to bond the analytes (A) of the sample portions (P1, P2, P3) to corresponding capture molecules (M),
wherein after the analytes (A) have bonded to the corresponding capture molecules (M), the sample portions (P1, P2, P3) are carried away from the sensor arrangement sequentially, and
wherein the bonded analytes (A) of the sample portions (P1, P2, P3) are detected in a single or common detection process.

2. Method according to claim 1, **characterised in that** the sample (P) is divided between different reaction cavities (109) and/or **in that** the sample portions (P1, P2, P3) are fed to different reaction cavities (109).

3. Method according to claim 2, **characterised in that** the analytes (A) of the sample portions (P1, P2, P3) are amplified by means of amplification reactions, in particular PCR, in the different reaction cavities (109).

4. Method according to claim 3, **characterised in that** the sample portions (P1, P2, P3) are amplified in parallel and/or independently from one another.

5. Method according to any of the preceding claims, **characterised in that** the analytes (A) of the sample portions (P1, P2, P3) are bonded to capture molecules (M) of the sensor arrangement and/or sensor apparatus (113) and that the bonded analytes (A) are detected by means of the sensor arrangement and/or sensor apparatus (113).

6. Method according to claim 5, **characterised in that** the bonded analytes (A) are detected electrochemically and/or by redox cycling.

7. Method according to any of the preceding claims, **characterised in that** the sample portions (P1, P2, P3) are fed to the sensor arrangement in a first conveying direction (R1), in particular in order to bond the analytes (A) of the sample portions (P1, P2, P3) to the corresponding capture molecules (M).

8. Method according to any of the preceding claims, **characterised in that**, in particular after the analytes (A) have bonded to the corresponding capture molecules (M), the sample portions (P1, P2, P3) are carried away from the sensor arrangement in a second conveying direction (R2) which is opposite to the first conveying direction (R1), in particular in order to collect the sample portions (P1, P2, P3) in a collection cavity (111).

9. Method according to any of the preceding claims, **characterised in that** the sample portions (P1, P2, P3) and a pretreatment fluid, in particular a reagent and/or wash buffer, are fed to the sensor arrangement from different sides.

10. Method according to any of the preceding claims, **characterised in that** the sample portions (P1, P2, P3) and/or a fluid for pretreatment, in particular a reagent and/or wash buffer, is/are conveyed from the sensor arrangement to a common collection cavity (111) of the cartridge (100), in particular in the second conveying direction (R2).

11. Method according to any of the preceding claims, **characterised in that**, in particular after the analytes (A) have bonded to the corresponding capture molecules (M) and/or before the bonded analytes (A) have been detected, the sensor arrangement is pretreated and/or flushed with a fluid, in particular a wash buffer and/or a reagent, for the detection.

12. Method according to any of the preceding claims, **characterised in that** the sensor arrangement is flushed with a wash buffer and/or is loaded with detector molecules (D) and/or a substrate (SU) for detecting the bonded analytes (A).

13. Method according to any of the preceding claims, **characterised in that** the sensor arrangement is flushed with the wash buffer multiple times, in particular after and/or during a plurality of method steps.

14. Method according to any of the preceding claims, **characterised in that** the analytes (A) of the sample portions (P1, P2, P3) are sequentially and/or individually bonded to the corresponding capture molecules (M) of a sensor apparatus (113), and the bonded analytes (A) of all the sample portions (P1, P2, P3) are identified, detected or determined together and/or in a single or common detection process.

15. Method according to any of the preceding aspects, **characterised in that** the cartridge (100) containing the sample (P) is received at least in part by an analysis device (200), the analysis device (200) preferably being pneumatically, thermally and/or electrically connected to the cartridge (100), and/or **in that** nucleic-acid sequences or proteins are detected as analytes (A) of the sample (P) or sample portions (P1, P2, P3).
